# EUROPEAN PATENT APPLICATION

(11) **EP 3 769 816 A1**
(43) Date of publication of application: **27.01.2021**
(21) Application number: 19188360.2
(22) Date of filing: 25.07.2019
(51) Int. Cl.: A61P 35/00, A61P 35/02, C07K 14/705, C07K 14/725, A61K 35/17, A61K 39/00, C07K 16/28

(54) **CAR-CD123 VECTOR AND USES THEREOF**

(71) Applicant: Ospedale Pediatrico Bambino Gesù, 00165 Roma (IT)
(72) Inventor: Quintarelli, Concetta, 00165 Roma (IT); De Angelis, Biagio, 00165 Roma (IT); Locatelli, Franco, 00165 Roma (IT)
(74) Representative: Capasso, Olga

(57) **Abstract**

The present invention relates to a chimeric antigen receptor (CAR) molecule comprising, from the N-terminus to the C-terminus: an extracellular domain and transmembrane domain of human CD19,
a) an antigen binding domain,
b) a spacer domain,
c) a transmembrane domain,
d) a cytoplasmatic domain,
preferably wherein the CAR is a CD123 specific CAR (CD123 CAR) and the antigen binding domain comprises VH and/or VL from a monoclonal anti-CD123 antibody, more preferably the antigen binding domain comprises a single chain variable fragment (scFv) that specifically binds to CD123.

SEQUENCE LISTING

<110>  Ospedale Pediatrico Bambino Gesù

<120>  CAR-CD123 vector and uses thereof

<130>  140950 BE

<160>  30

<170>  PatentIn version 3.5

<210>  1
<211>  333
<212>  PRT
<213>  Homo sapiens

<400>  1

Met Pro Pro Pro Arg Leu Leu Phe Phe Leu Leu Phe Leu Thr Pro Met
1               5                   10                  15

Glu Val Arg Pro Glu Glu Pro Leu Val Val Lys Val Glu Glu Gly Asp
            20                  25                  30

Asn Ala Val Leu Gln Cys Leu Lys Gly Thr Ser Asp Gly Pro Thr Gln
        35                  40                  45

Gln Leu Thr Trp Ser Arg Glu Ser Pro Leu Lys Pro Phe Leu Lys Leu
    50                  55                  60

Ser Leu Gly Leu Pro Gly Leu Gly Ile His Met Arg Pro Leu Ala Ile
65                  70                  75                  80

Trp Leu Phe Ile Phe Asn Val Ser Gln Gln Met Gly Gly Phe Tyr Leu
                85                  90                  95

Cys Gln Pro Gly Pro Pro Ser Glu Lys Ala Trp Gln Pro Gly Trp Thr
            100                 105                 110

Val Asn Val Glu Gly Ser Gly Glu Leu Phe Arg Trp Asn Val Ser Asp
        115                 120                 125

Leu Gly Gly Leu Gly Cys Gly Leu Lys Asn Arg Ser Ser Glu Gly Pro
    130                 135                 140

Ser Ser Pro Ser Gly Lys Leu Met Ser Pro Lys Leu Tyr Val Trp Ala
145                 150                 155                 160

Lys Asp Arg Pro Glu Ile Trp Glu Gly Glu Pro Pro Cys Leu Pro Pro
                165                 170                 175

Arg Asp Ser Leu Asn Gln Ser Leu Ser Gln Asp Leu Thr Met Ala Pro
            180                 185                 190

Gly Ser Thr Leu Trp Leu Ser Cys Gly Val Pro Pro Asp Ser Val Ser
        195                 200                 205

Arg Gly Pro Leu Ser Trp Thr His Val His Pro Lys Gly Pro Lys Ser
    210                 215                 220

Leu Leu Ser Leu Glu Leu Lys Asp Asp Arg Pro Ala Arg Asp Met Trp
225                 230                 235                 240

Val Met Glu Thr Gly Leu Leu Leu Pro Arg Ala Thr Ala Gln Asp Ala
                245                 250                 255

Gly Lys Tyr Tyr Cys His Arg Gly Asn Leu Thr Met Ser Phe His Leu
            260                 265                 270

Glu Ile Thr Ala Arg Pro Val Leu Trp His Trp Leu Leu Arg Thr Gly
        275                 280                 285

Gly Trp Lys Val Ser Ala Val Thr Leu Ala Tyr Leu Ile Phe Cys Leu
    290                 295                 300

Cys Ser Leu Val Gly Ile Leu His Leu Gln Arg Ala Leu Val Leu Arg
305                 310                 315                 320

Arg Lys Arg Lys Arg Met Thr Asp Pro Thr Arg Arg Phe
                325                 330


<210>  2
<211>  20
<212>  PRT
<213>  Artificial Sequence

<220>
<223>  2A peptide

<400>  2

Arg Gly Arg Gly Arg Gly Ser Leu Leu Thr Cys Gly Asp Val Glu Glu
1               5                   10                  15


Asn Pro Gly Pro
            20


<210>  3
<211>  19
<212>  PRT
<213>  Artificial Sequence

<220>
<223>  signal

<400>  3

Met Glu Phe Gly Leu Ser Trp Leu Phe Leu Val Ala Ile Leu Lys Gly
1               5                   10                  15

Val Gln Cys

<210>  4
<211>  114
<212>  PRT
<213>  Artificial Sequence

<220>
<223>  7G3 VL

<400>  4

Asp Phe Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Leu Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Tyr Thr Phe Gly Gly Gly Thr Lys Leu Glu Ile
            100                 105                 110

Lys Arg

<210>  5
<211>  118
<212>  PRT
<213>  Artificial Sequence

<220>
<223>  7G3 VH

<400>  5

Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Tyr
            20                  25                  30

Tyr Met Lys Trp Val Lys Gln Ser His Gly Lys Ser Leu Glu Trp Ile
        35                  40                  45

Gly Asp Ile Ile Pro Ser Asn Gly Ala Thr Phe Tyr Asn Gln Lys Phe
    50                  55                  60

Lys Gly Lys Ala Thr Leu Thr Val Asp Arg Ser Ser Ser Thr Ala Tyr
65                  70                  75                  80

Met His Leu Asn Ser Leu Thr Ser Glu Asp Ser Ala Val Tyr Tyr Cys
                85                  90                  95

Thr Arg Ser His Leu Leu Arg Ala Ser Trp Phe Ala Tyr Trp Gly Gln
            100                 105                 110

Gly Thr Leu Val Thr Val
        115

<210>  6
<211>  16
<212>  PRT
<213>  Artificial Sequence

<220>
<223>  trackable marker

<400>  6

Glu Leu Pro Thr Gln Gly Thr Phe Ser Asn Val Ser Thr Asn Val Ser
1               5                   10                  15

<210>  7
<211>  42
<212>  PRT
<213>  Artificial Sequence

<220>
<223>  spacer

<400>  7

Pro Ala Pro Arg Pro Pro Thr Pro Ala Pro Thr Ile Ala Ser Gln Pro
1               5                   10                  15

Leu Ser Leu Arg Pro Glu Ala Cys Arg Pro Ala Ala Gly Gly Ala Val

20                  25                  30

His Thr Arg Gly Leu Asp Phe Ala Cys Asp
        35                  40

<210>  8
<211>  21
<212>  PRT
<213>  Homo sapiens

<400>  8

Ile Tyr Ile Trp Ala Pro Leu Ala Gly Thr Cys Gly Val Leu Leu Leu
1               5                   10                  15

Ser Leu Val Ile Thr
            20

<210>  9
<211>  16
<212>  PRT
<213>  Homo sapiens

<400>  9

Leu Tyr Cys Asn His Arg Asn Arg Arg Arg Val Cys Lys Cys Pro Arg
1               5                   10                  15

<210>  10
<211>  42
<212>  PRT
<213>  Homo sapiens

<400>  10

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  11
<211>  112
<212>  PRT
<213>  Homo sapiens

<400>  11

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  12
<211>  869
<212>  PRT
<213>  Artificial Sequence

<220>
<223>  deltaCD19-2A-CAR.CD123-4.1BB-zeta CAR

<400>  12

Met Pro Pro Pro Arg Leu Leu Phe Phe Leu Leu Phe Leu Thr Pro Met
1               5                   10                  15

Glu Val Arg Pro Glu Glu Pro Leu Val Val Lys Val Glu Glu Gly Asp
            20                  25                  30

Asn Ala Val Leu Gln Cys Leu Lys Gly Thr Ser Asp Gly Pro Thr Gln
        35                  40                  45

Gln Leu Thr Trp Ser Arg Glu Ser Pro Leu Lys Pro Phe Leu Lys Leu
    50                  55                  60

Ser Leu Gly Leu Pro Gly Leu Gly Ile His Met Arg Pro Leu Ala Ile
65                  70                  75                  80

Trp Leu Phe Ile Phe Asn Val Ser Gln Gln Met Gly Gly Phe Tyr Leu
                85                  90                  95

Cys Gln Pro Gly Pro Pro Ser Glu Lys Ala Trp Gln Pro Gly Trp Thr
            100                 105                 110

Val Asn Val Glu Gly Ser Gly Glu Leu Phe Arg Trp Asn Val Ser Asp

115                 120                 125

Leu Gly Gly Leu Gly Cys Gly Leu Lys Asn Arg Ser Ser Glu Gly Pro
    130                 135                 140

Ser Ser Pro Ser Gly Lys Leu Met Ser Pro Lys Leu Tyr Val Trp Ala
145                 150                 155                 160

Lys Asp Arg Pro Glu Ile Trp Glu Gly Glu Pro Pro Cys Leu Pro Pro
                165                 170                 175

Arg Asp Ser Leu Asn Gln Ser Leu Ser Gln Asp Leu Thr Met Ala Pro
            180                 185                 190

Gly Ser Thr Leu Trp Leu Ser Cys Gly Val Pro Pro Asp Ser Val Ser
        195                 200                 205

Arg Gly Pro Leu Ser Trp Thr His Val His Pro Lys Gly Pro Lys Ser
    210                 215                 220

Leu Leu Ser Leu Glu Leu Lys Asp Asp Arg Pro Ala Arg Asp Met Trp
225                 230                 235                 240

Val Met Glu Thr Gly Leu Leu Leu Pro Arg Ala Thr Ala Gln Asp Ala
                245                 250                 255

Gly Lys Tyr Tyr Cys His Arg Gly Asn Leu Thr Met Ser Phe His Leu
            260                 265                 270

Glu Ile Thr Ala Arg Pro Val Leu Trp His Trp Leu Leu Arg Thr Gly
        275                 280                 285

Gly Trp Lys Val Ser Ala Val Thr Leu Ala Tyr Leu Ile Phe Cys Leu
    290                 295                 300

Cys Ser Leu Val Gly Ile Leu His Leu Gln Arg Ala Leu Val Leu Arg
305                 310                 315                 320

Arg Lys Arg Lys Arg Met Thr Asp Pro Thr Arg Arg Phe Arg Gly Arg
                325                 330                 335

Gly Arg Gly Ser Leu Leu Thr Cys Gly Asp Val Glu Glu Asn Pro Gly
            340                 345                 350

Pro Met Glu Phe Gly Leu Ser Trp Leu Phe Leu Val Ala Ile Leu Lys
        355                 360                 365

Gly Val Gln Cys Ser Arg Asp Phe Val Met Thr Gln Ser Pro Ser Ser
    370                 375                 380

Leu Thr Val Thr Ala Gly Glu Lys Val Thr Met Ser Cys Lys Ser Ser
385                 390                 395                 400

Gln Ser Leu Leu Asn Ser Gly Asn Gln Lys Asn Tyr Leu Thr Trp Tyr
                405                 410                 415

Leu Gln Lys Pro Gly Gln Pro Pro Lys Leu Leu Ile Tyr Trp Ala Ser
            420                 425                 430

Thr Arg Glu Ser Gly Val Pro Asp Arg Phe Thr Gly Ser Gly Ser Gly
        435                 440                 445

Thr Asp Phe Thr Leu Thr Ile Ser Ser Val Gln Ala Glu Asp Leu Ala
    450                 455                 460

Val Tyr Tyr Cys Gln Asn Asp Tyr Ser Tyr Pro Tyr Thr Phe Gly Gly
465                 470                 475                 480

Gly Thr Lys Leu Glu Ile Lys Arg Gly Gly Gly Ser Gly Gly Gly Gly
                485                 490                 495

Glu Val Gln Leu Gln Gln Ser Gly Pro Glu Leu Val Lys Pro Gly Ala
            500                 505                 510

Ser Val Lys Met Ser Cys Lys Ala Ser Gly Tyr Thr Phe Thr Asp Tyr
        515                 520                 525

Tyr Met Lys Trp Val Lys Gln Ser His Gly Lys Ser Leu Glu Trp Ile
    530                 535                 540

Gly Asp Ile Ile Pro Ser Asn Gly Ala Thr Phe Tyr Asn Gln Lys Phe
545                 550                 555                 560

Lys Gly Lys Ala Thr Leu Thr Val Asp Arg Ser Ser Ser Thr Ala Tyr
                565                 570                 575

Met His Leu Asn Ser Leu Thr Ser Glu Asp Ser Ala Val Tyr Tyr Cys
            580                 585                 590

Thr Arg Ser His Leu Leu Arg Ala Ser Trp Phe Ala Tyr Trp Gly Gln
        595                 600                 605

Gly Thr Leu Val Thr Val Ser Ala Gly Ser Glu Leu Pro Thr Gln Gly
    610                 615                 620

Thr Phe Ser Asn Val Ser Thr Asn Val Ser Pro Ala Pro Arg Pro Pro
625                 630                 635                 640

Thr Pro Ala Pro Thr Ile Ala Ser Gln Pro Leu Ser Leu Arg Pro Glu
                645                 650                 655

Ala Cys Arg Pro Ala Ala Gly Gly Ala Val His Thr Arg Gly Leu Asp
            660                 665                 670

Phe Ala Cys Asp Ile Tyr Ile Trp Ala Pro Leu Ala Gly Thr Cys Gly
        675                 680                 685

Val Leu Leu Leu Ser Leu Val Ile Thr Leu Tyr Cys Asn His Arg Asn
    690                 695                 700

Arg Arg Arg Val Cys Lys Cys Pro Arg Val Asp Lys Arg Gly Arg Lys
705                 710                 715                 720

Lys Leu Leu Tyr Ile Phe Lys Gln Pro Phe Met Arg Pro Val Gln Thr
                725                 730                 735

Thr Gln Glu Glu Asp Gly Cys Ser Cys Arg Phe Pro Glu Glu Glu Glu
            740                 745                 750

Gly Gly Cys Glu Leu Arg Val Lys Phe Ser Arg Ser Ala Asp Ala Pro
        755                 760                 765

Ala Tyr Gln Gln Gly Gln Asn Gln Leu Tyr Asn Glu Leu Asn Leu Gly
    770                 775                 780

Arg Arg Glu Glu Tyr Asp Val Leu Asp Lys Arg Arg Gly Arg Asp Pro
785                 790                 795                 800

Glu Met Gly Gly Lys Pro Arg Arg Lys Asn Pro Gln Glu Gly Leu Tyr
                805                 810                 815

Asn Glu Leu Gln Lys Asp Lys Met Ala Glu Ala Tyr Ser Glu Ile Gly
            820                 825                 830

Met Lys Gly Glu Arg Arg Arg Gly Lys Gly His Asp Gly Leu Tyr Gln
        835                 840                 845

Gly Leu Ser Thr Ala Thr Lys Asp Thr Tyr Asp Ala Leu His Met Gln
    850                 855                 860

Ala Leu Pro Pro Arg
865

<210>  13
<211>  999
<212>  DNA
<213>  Homo sapiens

<400>  13
atgccaccac ctcgcctgct gttctttctg ctgttcctga cacctatgga ggtgcgacct      60

gaggaaccac tggtcgtgaa ggtcgaggaa ggcgacaatg ccgtgctgca gtgcctgaaa     120

ggcacttctg atgggccaac tcagcagctg acctggtcca gggagtctcc cctgaagcct     180

tttctgaaac tgagcctggg actgccagga ctgggaatcc acatgcgccc tctggctatc     240

tggctgttca tcttcaacgt gagccagcag atgggaggat tctacctgtg ccagccagga     300

ccaccatccg agaaggcctg gcagcctgga tggaccgtca acgtggaggg gtctggagaa     360

ctgtttaggt ggaatgtgag tgacctggga ggactgggat gtgggctgaa gaaccgctcc     420

tctgaaggcc caagttcacc ctcagggaag ctgatgagcc caaaactgta cgtgtgggcc     480

aaagatcggc ccgagatctg ggagggagaa cctccatgcc tgccacctag agacagcctg     540

aatcagagtc tgtcacagga tctgacaatg gcccccgggt ccactctgtg gctgtcttgt     600

ggagtcccac ccgacagcgt gtccagaggc cctctgtcct ggacccacgt gcatcctaag     660

gggccaaaaa gtctgctgtc actggaactg aaggacgatc ggcctgccag agacatgtgg     720

gtcatggaga ctggactgct gctgccacga gcaaccgcac aggatgctgg aaaatactat     780

tgccaccggg gcaatctgac aatgtccttc catctggaga tcactgcaag gcccgtgctg     840

tggcactggc tgctgcgaac cggaggatgg aaggtcagtg ctgtgacact ggcatatctg     900

atcttttgcc tgtgctccct ggtgggcatt ctgcatctgc agagagccct ggtgctgcgg     960

agaaagagaa agagaatgac tgacccaaca agaaggttt                            999


<210>  14
<211>  60
<212>  DNA
<213>  Artificial Sequence

<220>
<223>  2A peptide

<400>  14
cgcggccgcg gccgagggag cctgctgaca tgtggcgatg tggaggaaaa cccaggacca      60


<210>  15
<211>  63
<212>  DNA
<213>  Artificial Sequence

<220>
<223>  signal peptide

<400>  15
atggagtttg gactttcttg gttgtttttg gtggcaattc tgaagggtgt ccagtgtagc      60

agg                                                                    63


<210>  16
<211>  342
<212>  DNA
<213>  Artificial Sequence

<220>
<223>  7G3 VL

<400>  16
gactttgtaa tgacccaatc tccaagctct cttacggtaa cggcaggaga gaaagtcacc      60

atgtcatgta aatccagtca atccctcttg aactcaggca accagaaaaa ttatcttacg     120

tggtatcttc aaaagccggg gcaaccccca aaactcctga tctactgggc atcaaccagg     180

gagtccggcg tccccgaccg ctttacgggt agtggaagtg gaaccgattt tacccttact     240

atcagcagcg tacaagcgga agacttggct gtgtattatt gtcaaaatga ttattcatat     300

ccctatactt tcggtggagg gactaaactt gaaattaaac ga                        342


<210>  17
<211>  24
<212>  DNA
<213>  Artificial Sequence

<220>
<223>  flex

<400>  17
ggcggaggaa gcggaggtgg gggc                                             24


<210>  18
<211>  354
<212>  DNA
<213>  Artificial Sequence

<220>
<223>  VH 7G3

<400>  18
gaagtccagt tgcaacaatc tggccctgag ttggtaaagc ccggagcctc tgtgaagatg      60

agttgtaagg cttcagggta tacatttaca gactattata tgaaatgggt caaacaatct     120

cacggtaaat ccttggagtg gattggcgat attatcccga gtaacggtgc cacgttctac     180

aaccagaagt tcaagggcaa ggcaacactg acggtagacc gcagcagcag cacggcgtat     240

atgcacctga actcattgac ttcagaggat agtgcagttt actactgtac tcggagtcat     300

ttgctgagag cgagttggtt cgcctattgg ggtcagggca cactcgttac cgta           354


<210>  19

<211>  12
<212>  DNA
<213>  Artificial Sequence

<220>
<223>  link

<400>  19
tctgcaggat cc                                                          12


<210>  20
<211>  48
<212>  DNA
<213>  Homo sapiens

<400>  20
gaacttccta ctcaggggac tttctcaaac gttagcacaa acgtaagt                   48


<210>  21
<211>  21
<212>  DNA
<213>  Artificial Sequence

<220>
<223>  spacer

<400>  21
   cccgccccaa gaccccccac a                                                 21


<210>  22
<211>  126
<212>  DNA
<213>  Homo sapiens

<400>  22
cccgccccaa gaccccccac acctgcgccg accattgctt ctcaacccct gagtttgaga       60

cccgaggcct gccggccagc tgccggcggg gccgtgcata caagaggact cgatttcgct      120

tgcgac                                                                 126


<210>  23
<211>  63
<212>  DNA
<213>  Homo sapiens

<400>  23
atctatatct gggcacctct cgctggcacc tgtggagtcc ttctgctcag cctggttatt      60

act                                                                    63


<210>  24
<211>  48
<212>  DNA
<213>  Homo sapiens

<400>  24
ctgtactgta atcaccggaa tcgccgccgc gtttgtaagt gtcccagg                   48

<210>  25
<211>  126
<212>  DNA
<213>  Homo sapiens

<400>  25
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126


<210>  26
<211>  339
<212>  DNA
<213>  Homo sapiens

<400>  26
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgctag                            339


<210>  27
<211>  8
<212>  PRT
<213>  Artificial Sequence

<220>
<223>  linker

<400>  27

Gly Gly Gly Ser Gly Gly Gly Gly
1               5


<210>  28
<211>  4
<212>  PRT
<213>  Artificial Sequence

<220>
<223>  linker

<400>  28

Ser Ala Gly Ser
1


<210>  29

<211>  2610
<212>  DNA
<213>  Artificial Sequence

<220>
<223>  nucleotide sequence encoding CD123 chimeric antigen receptor

<400>  29
atgccaccac ctcgcctgct gttctttctg ctgttcctga cacctatgga ggtgcgacct      60

gaggaaccac tggtcgtgaa ggtcgaggaa ggcgacaatg ccgtgctgca gtgcctgaaa     120

ggcacttctg atgggccaac tcagcagctg acctggtcca gggagtctcc cctgaagcct     180

tttctgaaac tgagcctggg actgccagga ctgggaatcc acatgcgccc tctggctatc     240

tggctgttca tcttcaacgt gagccagcag atgggaggat tctacctgtg ccagccagga     300

ccaccatccg agaaggcctg gcagcctgga tggaccgtca acgtggaggg gtctggagaa     360

ctgtttaggt ggaatgtgag tgacctggga ggactgggat gtgggctgaa gaaccgctcc     420

tctgaaggcc caagttcacc ctcagggaag ctgatgagcc caaaactgta cgtgtgggcc     480

aaagatcggc ccgagatctg ggagggagaa cctccatgcc tgccacctag agacagcctg     540

aatcagagtc tgtcacagga tctgacaatg gcccccgggt ccactctgtg gctgtcttgt     600

ggagtcccac ccgacagcgt gtccagaggc cctctgtcct ggacccacgt gcatcctaag     660

gggccaaaaa gtctgctgtc actggaactg aaggacgatc ggcctgccag agacatgtgg     720

gtcatggaga ctggactgct gctgccacga gcaaccgcac aggatgctgg aaaatactat     780

tgccaccggg gcaatctgac aatgtccttc catctggaga tcactgcaag gcccgtgctg     840

tggcactggc tgctgcgaac cggaggatgg aaggtcagtg ctgtgacact ggcatatctg     900

atcttttgcc tgtgctccct ggtgggcatt ctgcatctgc agagagccct ggtgctgcgg     960

agaaagagaa agagaatgac tgacccaaca agaaggtttc gcggccgcgg ccgagggagc    1020

ctgctgacat gtggcgatgt ggaggaaaac ccaggaccaa tggagtttgg actttcttgg    1080

ttgtttttgg tggcaattct gaagggtgtc cagtgtagca gggactttgt aatgacccaa    1140

tctccaagct ctcttacggt aacggcagga gagaaagtca ccatgtcatg taaatccagt    1200

caatccctct tgaactcagg caaccagaaa aattatctta cgtggtatct tcaaaagccg    1260

gggcaacccc caaaactcct gatctactgg gcatcaacca gggagtccgg cgtccccgac    1320

cgctttacgg gtagtggaag tggaaccgat tttaccctta ctatcagcag cgtacaagcg    1380

gaagacttgg ctgtgtatta ttgtcaaaat gattattcat atccctatac tttcggtgga    1440

gggactaaac ttgaaattaa acgaggcgga ggaagcggag gtgggggcga agtccagttg    1500

caacaatctg gccctgagtt ggtaaagccc ggagcctctg tgaagatgag ttgtaaggct    1560

tcagggtata catttacaga ctattatatg aaatgggtca aacaatctca cggtaaatcc    1620

ttggagtgga ttggcgatat tatcccgagt aacggtgcca cgttctacaa ccagaagttc    1680

aagggcaagg caacactgac ggtagaccgc agcagcagca cggcgtatat gcacctgaac 1740

tcattgactt cagaggatag tgcagtttac tactgtactc ggagtcattt gctgagagcg 1800

agttggttcg cctattgggg tcagggcaca ctcgttaccg tatctgcagg atccgaactt 1860

cctactcagg ggactttctc aaacgttagc acaaacgtaa gtcccgcccc aagacccccc 1920

acacctgcgc cgaccattgc ttctcaaccc ctgagtttga gacccgaggc ctgccggcca 1980

gctgccggcg gggccgtgca tacaagagga ctcgatttcg cttgcgacat ctatatctgg 2040

gcacctctcg ctggcacctg tggagtcctt ctgctcagcc tggttattac tctgtactgt 2100

aatcaccgga atcgccgccg cgtttgtaag tgtcccaggg tcgacaaacg gggcagaaag 2160

aaactcctgt atatattcaa acaaccattt atgagaccag tacaaactac tcaagaggaa 2220

gatggctgta gctgccgatt tccagaagaa gaagaaggag gatgtgaact gagagtgaag 2280

ttcagcagga gcgcagacgc ccccgcgtac cagcagggcc agaaccagct ctataacgag 2340

ctcaatctag gacgaagaga ggagtacgat gttttggaca agagacgtgg ccgggaccct 2400

gagatggggg gaaagccgag aaggaagaac cctcaggaag gcctgtacaa tgaactgcag 2460

aaagataaga tggcggaggc ctacagtgag attgggatga aaggcgagcg ccggaggggc 2520

aaggggcacg atggccttta ccagggtctc agtacagcca ccaaggacac ctacgacgcc 2580

cttcacatgc aggccctgcc ccctcgctag 2610

<210> 30
<211> 240
<212> PRT
<213> Artificial Sequence

<220>
<223> scFv

<400> 30

Asp Phe Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Leu Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Tyr Thr Phe Gly Gly Gly Thr Lys Leu Glu Ile
            100                 105                 110

Lys Arg Gly Gly Gly Ser Gly Gly Gly Gly Glu Val Gln Leu Gln Gln
        115                 120                 125

Ser Gly Pro Glu Leu Val Lys Pro Gly Ala Ser Val Lys Met Ser Cys
    130                 135                 140

Lys Ala Ser Gly Tyr Thr Phe Thr Asp Tyr Tyr Met Lys Trp Val Lys
145                 150                 155                 160

Gln Ser His Gly Lys Ser Leu Glu Trp Ile Gly Asp Ile Ile Pro Ser
                165                 170                 175

Asn Gly Ala Thr Phe Tyr Asn Gln Lys Phe Lys Gly Lys Ala Thr Leu
            180                 185                 190

Thr Val Asp Arg Ser Ser Ser Thr Ala Tyr Met His Leu Asn Ser Leu
        195                 200                 205

Thr Ser Glu Asp Ser Ala Val Tyr Tyr Cys Thr Arg Ser His Leu Leu
    210                 215                 220

Arg Ala Ser Trp Phe Ala Tyr Trp Gly Gln Gly Thr Leu Val Thr Val
225                 230                 235                 240

## Description

### TECHNICAL FIELD

The present invention concerns the generation of a novel chimeric antigen receptor (CAR)-CD123 vector for treatment of CD123+ tumors such as Acute Myeloid Leukemia (AML) [1], B-lymphoid leukemias, blastic plasmacytoid dendritic neoplasms (BPDCN) [2], myelodysplastic syndrome [3] and hairy cell leukemia [4].

### BACKGROUND ART

Acute myeloid leukemia (AML) is a heterogeneous clonal disorder characterized by the accumulation of abnormal immature blasts. The standard upfront chemotherapy AML treatment remains unchanged despite the advancement in understanding the leukemic biology. The current induction treatment- also called remission induction therapy- has the goal to clear the blood and bone marrow of immature blasts and bring about a complete remission. This treatment is usually given over 1 week and it can produce initial complete remission in almost 70% of young adult patients. However, 43% of patients will eventually relapse, and 18% never attain a complete remission at frontline induction treatment [5]. Immunotherapy with T-cells genetically modified to express CARs represents an innovative approach for AML.

In the last years, great interest has been focused in the identification of surface molecules that are preferentially expressed by AML cells and leukemia stem cells (LSCs), in order to selectively target the tumor population while avoiding to affect the normal counterpart of hematopoietic stem/progenitor cells (HSPCs) [6].

CD123 is a glycoprotein of 360 amino acids also known as the transmembrane alpha subunit of the interleukin-3 receptor (IL-3Rα). Together with CD131, CD123 forms a high affinity IL-3R. Upon binding of IL-3, IL-3R promotes cell proliferation and survival [7].

The basal expression of CD123 is low to negligible in HSPCs, monocytes, a subset of dendritic cells (DC), named plasmacytoid DC - pDC [8] and endothelial cells [7] [9]. In contrast to the low expression on HSPCs, AML blasts widely overexpress CD123 [10] [11]. Moreover, CD123 overexpression on AML cells is associated with resistance to apoptosis, higher proliferating potential and poor prognosis [11] [12] [13].

Notably, CD123 is selectively overexpressed not only by AML blasts, but also by a quiescent population of AML LSCs, which has been shown to be one of the major players in chemotherapy drug resistance [14]. The differential expression of CD123 on normal HSCs and LSCs makes CD123 an attractive target for AML treatment.

Moreover, CD123 can be used to delineate MDS stem cells in patients at high risk for MDS and that the CD123-positive population is biologically distinct from normal hematopoietic stem cells [3].

Several antibody-based therapies have been developed targeting CD123 [15] [16] [14]. To date, the CD123 targeting therapies have been shown to be safe with no major adverse effects reported on hematopoiesis. Their anti-leukemic activities in humans are still being investigated.

An alternative AML therapeutic approach utilizes T cells expressing a CAR that redirects T cell specificity towards CD123 in an MHC-independent manner [16]. CAR T cells have the potential to proliferate and persist in humans for many years establishing a long-term tumor immunity. The first generation of CARs consist of a single-chain variable fragment (scFv) from a monoclonal antibody (e.g. anti-CD123) fused to the signaling domain of CD3ζ in the typical structure scFv-spacer-CD3z. In addition to the structure just described, the second-generation CARs have one costimulatory endodomain (e.g. CD28, or 41BB) that helps a) completing the T activation and b) avoiding apoptosis by promoting IL2 secretion.

Preclinical studies using second generation of CAR-T cells with anti-CD123- CD28ζ CAR and anti-CD123-41BBζ CAR T cells have demonstrated potent leukemia killing ability in vitro and in vivo however produced incongruous results regarding their myeloablative effect on healthy CD123+ cells [17] [18]. Multiple phase I trials (ClinicalTrials.gov number NCT03796390, NCT02937103, NCT03114670, NCT03672851) for CD123-directed CAR T cell therapy are currently underway to validate the effect and safety profiles.

Clearly, the anti-CD123 CAR T technology has great potential in the treatment of AML and other CD123+ tumors. While the benefits of incorporating at least one costimulatory domain to the CAR constructs are well established, the choice of scFv, and extracellular spacer domain, the T cell subsets, and the manufacturing process remain an area of intense research.

There is still the need for identifying a CAR T vector for the treatment of CD123+ tumors.

Moreover, CD123-redirected T cell treatment of mice engrafted with normal human hematopoietic cells resulted in profound myeloablation, raising concerns for hematologic toxicity in patients with AML who may be treated with such therapies.

### DETAILED DESCRIPTION OF THE INVENTION

Inventors hypothesized that the incorporation of a novel suicide gene in the CAR.CD123 approach could minimize this bystander toxicity without impairing leukaemia control, thereby increasing the therapeutic window of anti-leukaemia CAR T cell or CAR Innate cell immunotherapy. In the light of the above, the present invention provides a novel second-generation CAR-CD123.

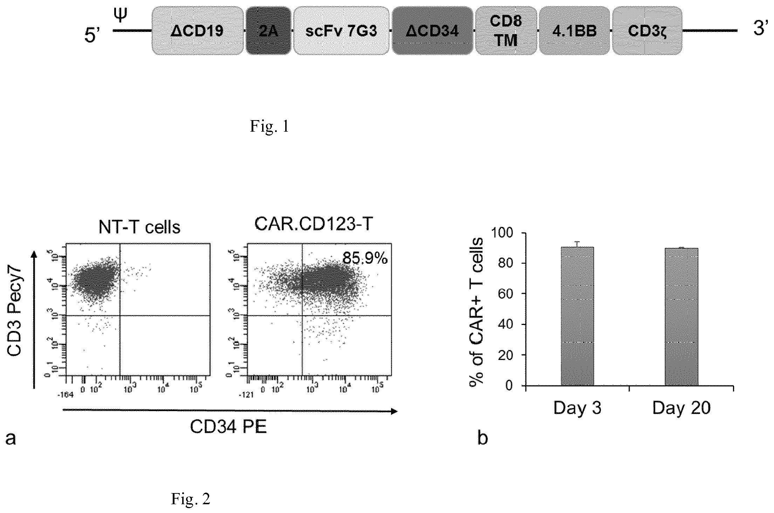

More specifically, the inventors constructed a CAR-CD123 structure (Figure 1): ΔCD19-2A-CAR-CD123-ΔCD34.CD8.41BB.CD3ζ (Table 1: OPBG-242 vector) retroviral vector.

Inventors have designed a bicistronic vector, allowing the simultaneous expression of two transgenes, namely ΔCD19 and CARCD123 (ΔCD19-2A-CAR-CD123-ΔCD34.CD8.41BB.CD3ζ). Clonal retroviral producer cell line generates retroviral supernatant characterized by high titer (using PCR analysis for vector presence in the supernatant).

ΔCD19 represents the extracellular domain of human CD19 linked to the transmembrane portion. It has a triple function to help:
1) the selection of the genetically modified cells by clinical grade microbeads;
2) the phenotypic characterization of the genetically modified cells;
3) as inducible suicide gene, it can be targeted by FDA/EMA approved anti-CD19 bite antibody (e.g. blinatumomab).

CAR molecule is based on the single chain of the fused VH-VL region of the monoclonal antibody 7G3 specific for the human antigen CD123, in frame with CD8 transmembrane domain and its endo domain, 4.1BB costimulatory domain and CD3æ cytoplasmic domain for the transduction of the activatory signal after antigen engagement. CAR construct was cloned in a retroviral vector after the gene cassette including the sequence of ΔCD19 through the use of a 2A sequence.

Thus, the functional and structural components important for ΔCD19-2A-CAR-CD123-ΔCD34.CD8.41BB.CD3zeta (here after **ΔCD19-2A-CAR.CD123-4.1BB-ζ)** expression and activity are summarized in Table 1, and listed in the following:
- 5' LTR - Retroviral long terminal repeat at 5' end of vector (functions as promoter sequence)
- ψ - Retroviral encapsidation signal (psi; necessary for packaging of RNA into virion particles)
- SA - splice acceptor site
- ΔCD19 consists of the optimized human (extracellular and transmembrane) domains of the CD 19 marker
- 2A - encodes a synthetic 20 amino acid peptide from Thosea Asigna insect virus, which functions as a cleavable linker between the ΔCD 19 protein and CAR proteins
- Signal peptide - short aminoacid sequence to allow the correct translocation of the secretory proteins from the Endoplasmic Reticulum to the cellular membrane.
- *ΔCD34 consists of a short peptide derived from human CD34, helping to detect CAR+ cells after transduction*
- CAR- CAR molecule based on the following elements indicated in the order of their location: single chain of the fused V_{H}-V_{L} region of the monoclonal antibody 7G3 specific for the human antigen CD123, in frame with ΔCD34 domain, CD8 domains (spacer, TM, CD8Cyt), 4.1BB costimulatory domain and CD3ζ cytoplasmic domain .
- 3' LTR - Retroviral long terminal repeat at 3' end of vector (functions as terminator/ polyadenylation sequences).

**Table 1: Functional Elements of OPBG-242 Plasmid**

| **Component** | **Start** | **End** |
|---|---|---|
| LTR | 399 | 999 |
| ΔCD19 | 2282 | 3280 |
| 2A sequence | 3281 | 3340 |
| Signal Peptide | 3341 | 3403 |
| SvFv 7G3 | 3404 | 3745 |
| 2A peptide | 3746 | 3769 |
| SvFv 7G3 | 3770 | 4123 |
| ΔCD34 | 4124 | 4183 |
| CD8 stalk (spacer) | 4184 | 4309 |
| CD8TM | 4310 | 4372 |
| CD8 Cyt | 4373 | 4420 |
| 4.1bb costimulation | 4427 | 4552 |
| CD3z | 4553 | 4891 |
| 3' LTR | 5069 | 5635 |
| Amp^{R} promoter | 6848 | 6952 |
| Amp^{R} | 6953 | 7813 |

Identity of the vector **ΔCD19-2A-CAR.CD123-4.1BB-ζ** :
A reference electronic vector sequence was assembled by combining the DNA sequence files for each component of the vector construct. Since the retroviral genome is RNA-based, sequence analysis was performed on the plasmid DNA used for transfection into the 293VEC cell line (initial step in retroviral product preparation). Bi-directional sequencing was performed by the inventors on the entire OPBG-242 vector. Sequencing runs were assembled using SnapGene software. No mismatched bases compared to the theoretical reference electronic sequence were identified.

According to the present invention, a novel CD123-specific chimeric antigen receptor (**ΔCD19-2A-CAR.CD123-4.1BB-ζ**) of second generation is now provided. Particularly, the following clinical grade second generation of CAR CD123 SFG retroviral vectors is provided:
**ΔCD19-2A-CAR.CD123-4.1BB-ζ** (ΔCD19-2A-CAR-CD123-ΔCD34.CD8.41BB.CD3ζ) composed by:
- ΔCD19 consists of the optimize human (extracellular and transmembrane) domains of the CD 19 marker;
- 2A peptide - encodes a synthetic 20 amino acid peptide from Thosea Asigna insect virus, which functions as a cleavable linker between the ΔCD19 protein and CAR proteins;
- A single chain variable fragment (scFv) from 7G3 hybridoma,
- A trackable marker CD34 derived epitope (ΔCD34) of only 16 amino acid (aa) (as trackable marker) for a rapid identification by FACS (Fluorescence-activated cell sorting) System and/or selection by Cell Sorter System of gene modified cells;
- A spacer represented by CD8 regions to avoid the immunogenic IgG4 Fc region.
- a transmembrane domain from the transmembrane domain of CD8 to improve molecule stabilization
- A small portion of CD8 cytoplasmatic portion to facilitate the signal from to single chain to 4.1BB-CD3 zeta chain (4.1BB-ζ)

A costimulatory domain was added to the **ΔCD19-2A-CAR.CD123-4.1BB-ζ** vector: 4-1BB fused to CD3-ζ chain.

**In ΔCD19-2A-CAR.CD123-4.1BB-ζ,** the native nucleotide sequence of the trackable marker, the costimulatory domains and the CD3-ζ chain were modified to obtain a codon optimization to improve the efficient protein expression.

**Table 2: Comparison among CD123 CARs. The table shows the differences between known CD123 CARs and the one object of the present invention (Retroviral OPBG).**

| **Platform** | **CAR Generation** | **Single chain** | **Suicide gene/selectable marker** | **trackable marker** | **Spacer** | **Transmembrane (TM)** | **Costimulatory domains** | **Reference** |
|---|---|---|---|---|---|---|---|---|
| **Lentiviral** | 2 | 26292 or 32716 | none | EGFRt | IgG4 Fc region | CD28 | CD28 | [17] |
| **Sleeping Beauty System** | 2 | 26292, 32701, 32703 and 32716 | none | IgG4 Fc region | CD8α/IgG 4 | CD28 | CD28 | [19] |
| **Retroviral** | 1 | 7G3 | none | IgG4 Fc region | IgG4 Fc region | CD28 | none | [14] |
| | | | | | | | | |
| **Retroviral (OPBG)** | 2 | **7G3** | **ΔCD19 (Codon optimized)** | **ΔCD34 (Codon optimized)** | **CD8 (Codon optimized)** | **CD8 (Codon optimized)** | **4.1BB (Codon optimized)** | **non-applicable** |

Therefore, it is an object of the present invention a CD123 chimeric antigen receptor molecule comprising or consisting of, from the N-terminus to the C- terminus:
a) **ΔCD19 extracellular and transmembrane domains,** such as: MPPPRLLFFLLFLTPMEVRPEEPLVVKVEEGDNAVLQCLKGTSDGPTQQLTWSRES PLKPFLKLSLGLPGLGIHMRPLAIWLFIFNVSQQMGGFYLCQPGPPSEKAWQPGWT VNVEGSGELFRWNVSDLGGLGCGLKNRSSEGPSSPSGKLMSPKLYVWAKDRPEIW EGEPPCLPPRDSLNQSLSQDLTMAPGSTLWLSCGVPPDSVSRGPLSWTHVHPKGPK SLLSLELKDDRPARDMWVMETGLLLPRATAQDAGKYYCHRGNLTMSFHLEITARP VLWHWLLRTGGWKVSAVTLAYLIFCLCSLVGILHLQRALVLRRKRKRMTDPTRRF (*SEQ ID NO:1*) (nucleotide ID NO: NM_001178098.2 and Protein ID NO: NP_001171569.1)
b) **2A peptide** - encodes a synthetic 20 amino acid peptide from Thosea Asigna insect virus, such as RGRGRGSLLTCGDVEENPGP (*SEQ ID NO:2*)
c) **a signal peptide,** such as MEFGLSWLFLVAILKGVQC (SEQ ID NO:3) (nucleotide ID NO: AB776838.1 and Protein ID NO: BAN63131.1), which is linked by a first linker (SR) as a connection sequence to:
d) **an anti CD123 single chain** antibody domain from 7G3 hybridoma comprising or consisting of the 7G3 VL sequence: DFVMTQSPSSLTVTAGEKVTMSCKSSQSLLNSGNQKNYLTWYLQKPGQPPKLLIY WASTRESGVPDRFTGSGSGTDFTLTISSVQAEDLAVYYCQNDYSYPYTFGGGTKLE IKR (SEQ ID NO:4) and linked by a second linker (GGGSGGGG (SEQ ID NO: 27)) to 7G3 VH sequence: EVQLQQSGPELVKPGASVKMSCKASGYTFTDYYMKWVKQSHGKSLEWIGDIIPSN GATFYNQKFKGKATLTVDRSSSTAYMHLNSLTSEDSAVYYCTRSHLLRASWFAY WGQGTLVTV(SEQ ID NO:5) said 7G3 VL and VH sequences being linked by a third linker (SAGS (SEQ ID NO: 28)) to:
e) **a trackable marker** consisting of ΔCD34: ELPTQGTFSNVSTNVS (SEQ ID NO:6) (nucleotide ID NO AB238231.1 and Protein ID NO: BAE46748.1)
f) **a spacer** chosen from the group consisting of extracellular portion of CD8α: PAPRPPTPAPTIASOPLSLRPEACRPAAGGAVHTRGLDFACD (SEQ ID NO: 7) (nucleotide ID NO: M12828.1 and Protein ID NO: AAB04637.1)
g) spacer CD8α which is mentioned above comprises the sequence PAPRPPTPAPT (aa. 1-11 of SEQ ID NO: 7) as a spacer
h) **a trans membrane domain consisting of CD8TM:** IYIWAPLAGTCGVLLLSLVIT (SEQ ID NO:8) (nucleotide ID NO NM_001768.6 and Protein ID NO: NP_001759.3)
i) **a citoplasmatic domain consisting of CD8a cyt:**
   LYCNHRNRRRVCKCPR (SEQ ID NO:9) (nucleotide ID NO NM_001768.6 and Protein ID NO: NP_001759.3); a linker of two amino acids, such as VD
j) **a co-stimulatory signalling domain CD137 (4-1BB)** sequence: KRGRKKLLYIFKQPFMRPVQTTQEEDGCSCRFPEEEEGGCEL (SEQ ID NO:10) (nucleotide ID NO: U03397.1 and Protein NO: AAA53133.1;
k) **and CD3-Zeta chain:** RVKFSRSADAPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQ ALPPR* (SEQ ID NO: 11) (nucleotide ID NO: J04132.1 And Protein ID: AAA60394.1)

According to a preferred embodiment of the present invention **ΔCD19-2A-CAR.CD123-4.1BB-ζ** chimeric antigen receptor molecule is:
MPPPRLLFFLLFLTPMEVRPEEPLVVKVEEGDNAVLQCLKGTSDGPTQQLTWSRESPLKPF LKLSLGLPGLGIHMRPLAIWLFIFNVSQQMGGFYLCQPGPPSEKAWQPGWTVNVEGSGEL FRWNVSDLGGLGCGLKNRSSEGPSSPSGKLMSPKLYVWAKDRPEIWEGEPPCLPPRDSLN QSLSQDLTMAPGSTLWLSCGVPPDSVSRGPLSWTHVHPKGPKSLLSLELKDDRPARDMW VMETGLLLPRATAQDAGKYYCHRGNLTMSFHLEITARPVLWHWLLRTGGWKVSAVTLA YLIFCLCSLVGILHLQRALVLRRKRKRMTDPTRRF**RGRGRGSLLTCGDVEENPGP**MEFG LSWLFLVAILKGVQCSRDFVMTQSPSSLTVTAGEKVTMSCKSSQSLLNSGNQKNYLTWYL QKPGQPPKLLIYWASTRESGVPDRFTGSGSGTDFTLTISSVQAEDLAVYYCQNDYSYPYTF GGGTKLEIKRGGGSGGGGEVQLQQSGPELVKPGASVKMSCKASGYTFTDYYMKWVKQS HGKSLEWIGDIIPSNGATFYNQKFKGKATLTVDRSSSTAYMHLNSLTSEDSAVYYCTRSHL LRASWFAYWGQGTLVTVSAGSELPTQGTFSNVSTNVSPAPRPPTPAPTIASQPLSLRPEACR PAAGGAVHTRGLDFACDIYIWAPLAGTCGVLLLSLVITLYCNHRNRRRVCKCPRVDKRGR KKLLYIFKQPFMRPVQTTQEEDGCSCRFPEEEEGGCELRVKFSRSADAPAYQQGQNQLYN ELNLGRREEYDVLDKRRGRDPEMGGKPRRKNPQEGLYNELQKDKMAEAYSEIGMKGER RRGKGHDGLYQGLSTATKDTYDALHMQALPPR* (SEQ ID NO: 12) Namely, this sequence, herewith named also as **ΔCD19-2A-CAR.CD123-4.1BB-ζ** .

The present invention concerns also a nucleotide sequence, which encodes CD123 chimeric antigen receptor described above.

According to an embodiment of the present invention, the nucleotide sequence is:

ATGCCACCACCTCGCCTGCTGTTCTTTCTGCTGTTCCTGACACCTATGGAGGTGCGACC
TGAGGAACCACTGGTCGTGAAGGTCGAGGAAGGCGACAATGCCGTGCTGCAGTGCCT
GAAAGGCACTTCTGATGGGCCAACTCAGCAGCTGACCTGGTCCAGGGAGTCTCCCCTG
AAGCCTTTTCTGAAACTGAGCCTGGGACTGCCAGGACTGGGAATCCACATGCGCCCTC
TGGCTATCTGGCTGTTCATCTTCAACGTGAGCCAGCAGATGGGAGGATTCTACCTGTG
CCAGCCAGGACCACCATCCGAGAAGGCCTGGCAGCCTGGATGGACCGTCAACGTGGA
GGGGTCTGGAGAACTGTTTAGGTGGAATGTGAGTGACCTGGGAGGACTGGGATGTGG
GCTGAAGAACCGCTCCTCTGAAGGCCCAAGTTCACCCTCAGGGAAGCTGATGAGCCCA
AAACTGTACGTGTGGGCCAAAGATCGGCCCGAGATCTGGGAGGGAGAACCTCCATGC
CTGCCACCTAGAGACAGCCTGAATCAGAGTCTGTCACAGGATCTGACAATGGCCCCCG
GGTCCACTCTGTGGCTGTCTTGTGGAGTCCCACCCGACAGCGTGTCCAGAGGCCCTCT
GTCCTGGACCCACGTGCATCCTAAGGGGCCAAAAAGTCTGCTGTCACTGGAACTGAAG
GACGATCGGCCTGCCAGAGACATGTGGGTCATGGAGACTGGACTGCTGCTGCCACGA
GCAACCGCACAGGATGCTGGAAAATACTATTGCCACCGGGGCAATCTGACAATGTCCT
TCCATCTGGAGATCACTGCAAGGCCCGTGCTGTGGCACTGGCTGCTGCGAACCGGAGG
ATGGAAGGTCAGTGCTGTGACACTGGCATATCTGATCTTTTGCCTGTGCTCCCTGGTGG
GCATTCTGCATCTGCAGAGAGCCCTGGTGCTGCGGAGAAAGAGAAAGAGAATGACTG
ACCCAACAAGAAGGTTTCGCGGCCGCGGCCGAGGGAGCCTGCTGACATGTGGCGATG
TGGAGGAAAACCCAGGACCAATGGAGTTTGGACTTTCTTGGTTGTTTTTGGTGGCAAT

TCTGAAGGGTGTCCAGTGTAGCAGGGACTTTGTAATGACCCAATCTCCAAGCTCTCTT
ACGGTAACGGCAGGAGAGAAAGTCACCATGTCATGTAAATCCAGTCAATCCCTCTTGA
ACTCAGGCAACCAGAAAAATTATCTTACGTGGTATCTTCAAAAGCCGGGCAACCCCC
AAAACTCCTGATCTACTGGGCATCAACCAGGGAGTCCGGCGTCCCCGACCGCTTTACG
GGTAGTGGAAGTGGAACCGATTTTACCCTTACTATCAGCAGCGTACAAGCGGAAGACT
TGGCTGTGTATTATTGTCAAAATGATTATTCATATCCCTATACTTTCGGTGGAGGGACT
AAACTTGAAATTAAACGAGGCGGAGGAAGCGGAGGTGGGGGCGAAGTCCAGTTGCAA
CAATCTGGCCCTGAGTTGGTAAAGCCCGGAGCCTCTGTGAAGATGAGTTGTAAGGCTT
CAGGGTATACATTTACAGACTATTATATGAAATGGGTCAAACAATCTCACGGTAAATC
CTTGGAGTGGATTGGCGATATTATCCCGAGTAACGGTGCCACGTTCTACAACCAGAAG
TTCAAGGGCAAGGCAACACTGACGGTAGACCGCAGCAGCAGCACGGCGTATATGCAC
CTGAACTCATTGACTTCAGAGGATAGTGCAGTTTACTACTGTACTCGGAGTCATTTGCT
GAGAGCGAGTTGGTTCGCCTATTGGGGTCAGGGCACACTCGTTACCGTATCTGCAGGA
TCCGAACTTCCTACTCAGGGGACTTTCTCAAACGTTAGCACAAACGTAAGTCCCGCCC
CAAGACCCCCCACACCTGCGCCGACCATTGCTTCTCAACCCCTGAGTTTGAGACCCGA
GGCCTGCCGGCCAGCTGCCGGCGGGGCCGTGCATACAAGAGGACTCGATTTCGCTTGC
GACATCTATATCTGGGCACCTCTCGCTGGCACCTGTGGAGTCCTTCTGCTCAGCCTGGT
TATTACTCTGTACTGTAATCACCGGAATCGCCGCCGCGTTTGTAAGTGTCCCAGGGTCG
ACAAACGGGGCAGAAAGAAaCTCCTGTATATATTCAAACAACCATTTATGAGACCAGT
ACAAACTACTCAAGAGGAAGATGGCTGTAGCTGCCGATTTCCAGAAGAAGAAGAAGG
AGGATGTGAACTGAGAGTGAAGTTCAGCAGGAGCGCAGACGCCCCCGCGTACCAGCA
GGGCCAGAACCAGCTCTATAACGAGCTCAATCTAGGACGAAGAGAGGAGTACGATGT
TTTGGACAAGAGACGTGGCCGGGACCCTGAGATGGGGGGAAAGCCGAGAAGGAAGA
ACCCTCAGGAAGGCCTGTACAATGAACTGCAGAAAGATAAGATGGCGGAGGCCTACA
GTGAGATTGGGATGAAAGGCGAGCGCCGGAGGGGCAAGGGGCACGATGGCCTTTACC
AGGGTCTCAGTACAGCCACCAAGGACACCTACGACGCCCTTCACATGCAGGCCCTGCC
CCCTCGCTAG (SEQ ID NO:29)

Namely, this nucleotide sequence, which encodes the sequence named also as **ΔCD19-2A-CAR.CD123-4.1BB-ζ,** comprises the following sequences:
**ΔCD19** 
ATGCCACCACCTCGCCTGCTGTTCTTTCTGCTGTTCCTGACACCTATGGAGGTGCGACC
TGAGGAACCACTGGTCGTGAAGGTCGAGGAAGGCGACAATGCCGTGCTGCAGTGCCT
 
GAAAGGCACTTCTGATGGGCCAACTCAGCAGCTGACCTGGTCCAGGGAGTCTCCCCTG
AAGCCTTTTCTGAAACTGAGCCTGGGACTGCCAGGACTGGGAATCCACATGCGCCCTC
TGGCTATCTGGCTGTTCATCTTCAACGTGAGCCAGCAGATGGGAGGATTCTACCTGTG
CCAGCCAGGACCACCATCCGAGAAGGCCTGGCAGCCTGGATGGACCGTCAACGTGGA
GGGGTCTGGAGAACTGTTTAGGTGGAATGTGAGTGACCTGGGAGGACTGGGATGTGG
GCTGAAGAACCGCTCCTCTGAAGGCCCAAGTTCACCCTCAGGGAAGCTGATGAGCCCA
AAACTGTACGTGTGGGCCAAAGATCGGCCCGAGATCTGGGAGGGAGAACCTCCATGC
CTGCCACCTAGAGACAGCCTGAATCAGAGTCTGTCACAGGATCTGACAATGGCCCCCG
GGTCCACTCTGTGGCTGTCTTGTGGAGTCCCACCCGACAGCGTGTCCAGAGGCCCTCT
GTCCTGGACCCACGTGCATCCTAAGGGGCCAAAAAGTCTGCTGTCACTGGAACTGAAG
GACGATCGGCCTGCCAGAGACATGTGGGTCATGGAGACTGGACTGCTGCTGCCACGA
GCAACCGCACAGGATGCTGGAAAATACTATTGCCACCGGGGCAATCTGACAATGTCCT
TCCATCTGGAGATCACTGCAAGGCCCGTGCTGTGGCACTGGCTGCTGCGAACCGGAGG
ATGGAAGGTCAGTGCTGTGACACTGGCATATCTGATCTTTTGCCTGTGCTCCCTGGTGG
GCATTCTGCATCTGCAGAGAGCCCTGGTGCTGCGGAGAAAGAGAAAGAGAATGACTG
ACCCAACAAGAAGGTTT(SEQ ID NO:13)
**2A peptide** 
CGCGGCCGCGGCCGAGGGAGCCTGCTGACATGTGGCGATGTGGAGGAAAACCCAGGA
CCA (SEQ ID NO:14)
**Signal peptide** 
ATGGAGTTTGGACTTTCTTGGTTGTTTTGGTGGCAATTCTGAAGGGTGTCCAGTGTAG
CAGG (SEQ ID NO:15)
**VL (7G3)** 
GACTTTGTAATGACCCAATCTCCAAGCTCTCTTACGGTAACGGCAGGAGAGAAAGTCA
CCATGTCATGTAAATCCAGTCAATCCCTCTTGAACTCAGGCAACCAGAAAAATTATCT
TACGTGGTATCTTCAAAAGCCGGGGCAACCCCCAAAACTCCTGATCTACTGGGCATCA
ACCAGGGAGTCCGGCGTCCCCGACCGCTTTACGGGTAGTGGAAGTGGAACCGATTTTA
CCCTTACTATCAGCAGCGTACAAGCGGAAGACTTGGCTGTGTATTATTGTCAAAATGA
TTATTCATATCCCTATACTTTCGGTGGAGGGACTAAACTTGAAATTAAACGA (SEQ ID
NO:16)
**Flex**
   GGCGGAGGAAGCGGAGGTGGGGGC (SEQ ID NO: 17)
**VH (7G3)** 
GAAGTCCAGTTGCAACAATCTGGCCCTGAGTTGGTAAAGCCCGGAGCCTCTGTGAAGA
TGAGTTGTAAGGCTTCAGGGTATACATTTACAGACTATTATATGAAATGGGTCAAACA
ATCTCACGGTAAATCCTTGGAGTGGATTGGCGATATTATCCCGAGTAACGGTGCCACG
TTCTACAACCAGAAGTTCAAGGGCAAGGCAACACTGACGGTAGACCGCAGCAGCAGC
ACGGCGTATATGCACCTGAACTCATTGACTTCAGAGGATAGTGCAGTTTACTACTGTA
CTCGGAGTCATTTGCTGAGAGCGAGTTGGTTCGCCTATTGGGGTCAGGGCACACTCGT
TACCGTA (SEQ ID NO:18)
**Link** (BamHl restriction site and connection sequence)
   TCTGCAGGATCC (SEQ ID NO:19)
**ΔCD34**
   GAACTTCCTACTCAGGGGACTTTCTCAAACGTTAGCACAAACGTAAGT (SEQ ID NO:20) **(AB238231.1)**
**Spacer** (to ameliorate the recognition by using anti-CD34 Ab)
   CCCGCCCCAAGACCCCCCACA (SEQ ID NO:21)
**Spacer (CD8a) extracellular** 
CCCGCCCCAAGACCCCCCACACCTGCGCCGACCATTGCTTCTCAACCCCTGAGTTTGA
GACCCGAGGCCTGCCGGCCAGCTGCCGGCGGGGCCGTGCATACAAGAGGACTCGATT
TCGCTTGCGAC (SEQ ID NO:22) (ID NO: M12828.1)
**CD8a (TM) transmembrane** 
ATCTATATCTGGGCACCTCTCGCTGGCACCTGTGGAGTCCTTCTGCTCAGCCTGGTTAT
TACT (SEQ ID NO:23) (ID NO: M12828.1)
**CD8a cytoplasmic (CD8a cyt)**
   CTGTACTGTAATCACCGGAATCGCCGCCGCGTTTGTAAGTGTCCCAGG (SEQ ID NO:24) (nucleotide ID NO **NM_001768.6**)
**Link of connection**
   GTCGAC
**4-1BB sequence** (a co-stimulatory signaling domain) 
AAACGGGGCAGAAAGAAaCTCCTGTATATATTCAAACAACCATTTATGAGACCAGTAC
AAACTACTCAAGAGGAAGATGGCTGTAGCTGCCGATTTCCAGAAGAAGAAGAAGGAG
GATGTGAACTG (SEQ ID NO:25) (nucleotide ID NO: U03397.1)
**CD3 Zeta chain**

AGAGTGAAGTTCAGCAGGAGCGCAGACGCCCCCGCGTACCAGCAGGGCCAGAACCAG
CTCTATAACGAGCTCAATCTAGGACGAAGAGAGGAGTACGATGTTTTGGACAAGAGA
CGTGGCCGGGACCCTGAGATGGGGGGAAAGCCGAGAAGGAAGAACCCTCAGGAAGG
CCTGTACAATGAACTGCAGAAAGATAAGATGGCGGAGGCCTACAGTGAGATTGGGAT
GAAAGGCGAGCGCCGGAGGGGCAAGGGGCACGATGGCCTTTACCAGGGTCTCAGTAC
AGCCACCAAGGACACCTACGACGCCCTTCACATGCAGGCCCTGCCCCCTCGCTAG
(SEQ ID NO:26) (nucleotide ID NO: J04132.1)

It is therefore an object of the invention a chimeric antigen receptor (CAR) molecule comprising, from the N-terminus to the C-terminus:
a) an extracellular domain and transmembrane domain of human CD19,
b) an antigen binding domain,
c) a spacer domain,
d) a transmembrane domain,
e) a cytoplasmatic domain,
preferably wherein the CAR is a CD123 specific CAR (CD123 CAR) and the antigen binding domain comprises VH and/or VL from a monoclonal anti-CD123 antibody, more preferably the antigen binding domain comprises a single chain variable fragment (scFv) that specifically binds to CD123.

It is included in the presen invention also a CAR molecule comprising at least one of the above elements a)-e). It is also included in the present invention, a CAR molecule wherein said elements are present in any order.

Preferably, in the CAR molecule according to the invention the extracellular and transmembrane domains of human CD19 comprise or consist of a sequence having at least 80% of identity to SEQ ID NO: 1 (MPPPRLLFFLLFLTPMEVRPEEPLVVKVEEGDNAVLQCLKGTSDGTQQLTWSRESPLKPFLKLSLGLPGLGIHMRPLAIWLFIFNVSQQMGGFYLCQPGPPSEKAWQPGWTVNVEGSGELFRWNVSDLGGLGCGLKNRSSEGPSSPSGKLMSPKLYVWAKDPEIWEGEPPCLPPRDSLNQSLSQDLTMAPGSTLWLSCGVPPDVSRGPLSWTHVHPKGPKSLLSELLKDDRPARDMWVMETGLLLPRATAQDAGGYYCHRGNLTMSFHLEITARPVLWHWLLRTGGWKVSAVTLAYLIFCLCSLVGILHLQRALVLRRKRKRMTDPTRRF), preferably it comprises or consists of SEQ ID NO: 1.

Preferably, the antigen binding domain comprises comprises a sequence having at least 80% of identity to the 7G3 VL sequence: DFVMTQSPSSLTVTAGEKVTMSCKSSQSLLNSGNQKNYLTWYLQKPGQPPKLLIYWASTR ESGVPDRFTGSGSGTDFTLTISSVQAEDLAVYYCQNDYSYPYTFGGGTKLEIKR (SEQ ID NO:4) and/or a sequence having at least 80% of identity to the 7G3: EVQLQQSGPELVKPGASVKMSCKASGYTFTDYYMKWVKQSHGKSLEWIGDIIPSNGATFY NQKFKGKATLTVDRSSSTAYMHLNSLTSEDSAVYYCTRSHLLRASWFAYWGQGTLVTV( SEQ ID NO:5), said VH and/or VL being optionally humanized, wherein said VH and VL are preferably separated by a first linker, more preferably said scFv comprises a VL comprising SEQ ID NO: 4 and a VH comprising SEQ ID NO: 5 .

Preferably the above antigen binding domain may comprise antigen binding fragments of the sequence above disclosed, e.g. at least one of the CDR comprised in the VH and VL sequences.

Said first linker preferably comprises or consists of the sequence GGGSGGGG (SEQ ID NO: 27) Said spacer preferably comprises the extacellular region of the CD8 alpha chain, preferably it comprises or consists of a sequence having at least 80% of identity to aa. 12-42 of SEQ ID NO: 7 (IASQPLSLRPEACRPAAGGAVHTRGLDFACD) and/or the spacer comprises or consists of a sequence having at least 80% of identity to aa. 1-11 of SEQ ID NO:7 (PAPRPPTPAPT), more preferably the spacer comprisises SEQ ID NO:7.

Preferably, said trans membrane domain comprises a trasmembrane domain of a protein selected from the group consiting of CD8, alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD15, preferably it comprises the trans membrane domain of a CD8, more preferably it comprises or consists of a sequence having at least 80% of identity to SEQ ID NO: 8 (IYIWAPLAGTCGVLLLSLVIT), even more preferably it comprises SEQ ID NO: 8.

Preferably, said cytoplasmatic domain comprises a region of CD8a cytoplasmatic portion and/or a 4-1BB co-stimulatory domain and/or a CD3-zeta chain. More preferably the cytoplasmatic domain comprises a region of CD8a cytoplasmatic portion and a 4-1BB co-stimulatory domain and a CD3-zeta chain. Preferably said cytoplasmatic domain comprises a linker between the CD8a cytoplasmic and the 4-1BB co-stimulatory domain. More preferably:
- the CD8a cytomplasmic comprises or consistis of a sequence having at least 80% of identity to SEQ ID NO:9 (LYCNHRNRRRVCKCPR), even more preferably it comprises SEQ ID NO:9, and/or
- the co-stimulatory signalling domain CD137 (4-1BB) comprises or consistis of a sequence having at least 80% of identity to SEQ ID NO:10 (KRGRKKLLYIFKQPFMRPVQTTQEEDGCSCRFPEEEEGGCEL), even more preferably it comprises SEQ ID NO: 10, and/or
- the CD3-Zeta chain comprises or consistis of a sequence having at least 80% of identity to SEQ ID NO: 11 (RVKFSRSADAPAYQQGQNQLYNELNLGRREEYDVLDKRRGRDPEMGGKPRRKNPQEGLYNELQKDKMAEYSEIGMKGERRRGKGHDGLYQGLSTATKDTYDALHMQALPPR*), even more preferably it comprises SEQ ID NO:11.

Preferably, the sequence encoding the linker between the CD8a cytomplasmic and the costimulatory signalling domain CD137 (4-1BB) consists of 6 nucleotides. More preferably it comprises or consists of a linker of two aminoacid, preferably VD.

In this included in the present invention a CAR molecule further comprising
- a clevable linker, preferably a 2A peptide or an IRES, and/or
- a signal peptide and/or
- a second linker, and/or
- a third linker and/or
- a trackable marker,
in any order.

In a preferred embodiment, the CAR molecule as above defined furter comprises:
i. between the extracellular domain and transmembrane domain of human CD19 and the antigen binding domain:
   - a clevable linker, preferably a 2A peptide or an IRES, and/or
   - a signal peptide and/or
   - a second linker, and/or
ii. between the antigen binding domain and the spacer domain.
   - a third linker and/or
   - a trackable marker.

Preferably the above clevable linker, signal peptide and the second linker, and the third linker and the trackable marker are present in the CAR molecule from the N-terminus to the C-terminus.

### More preferably:

- the 2 A peptide comprises or consists of a sequence having at least 80% of identity to SEQ ID NO:2 (RGRGRGSLLTCGDVEENPGP), more preferably it comprises SEQ ID NO:2, and/or
- the signal peptide comprises or consist of a sequence having at least 80% of identity to SEQ ID NO:3 (MEFGLSWLFLVAILKGVQC), more preferably it comprises SEQ ID NO:3, and/or
- the trackable marker comprises or consists of a sequence having at least 80% of identity to SEQ ID NO:6 (ELPTQGTFSNVSTNVS), more preferably it comprises SEQ ID NO:6.

Preferably, the second linker between the signal peptide and the antigen binding domain is encoded by a sequence of 6 nucleotides, preferably the linker comprises or consists of the aa. SR.

Preferably the third linker between the antigen binding domain and the trackable marker comprises or consists of the sequence SAGS (SEQ ID NO: 28).

Preferably, the seequence encoding for at least one of: the extracellular domain and transmembrane domain of human CD19, the trackable marker, the spacer domain, the transmembrane domain and the co-stimulatory domain is codon optimized.

In a preferred embodiment, the CAR molecule as above defined comprises:
from the N-terminus to the C-terminus:
a) an extracellular domain and transmembrane domain of human CD19, as above defined,
b) a clevable linker, as above defined
c) a signal peptide, as above defined
d) an antigen binding domain, as above defined
e) a trackable marker, as above defined
f) a spacer domain, as above defined
g) a transmembrane domain, as above defined
h) a cytoplasmatic domain, as above defined.

Preferably the CAR molecule comprises or consists of a sequence having at least 80% of identity to SEQ ID NO:12 (MPPPRLLFFLLFLTPMEVRPEEPLVVKVEEGDNAVLQCLKGTSDGPTQQLTWSRESPLKPF LKLSLGLPGLGIHMRPLAIWLFIFNVSQQMGGFYLCQPGPPSEKAWQPGWTVNVEGSGEL FRWNVSDLGGLGCGLKNRSSEGPSSPSGKLMSPKLYVWAKDRPEIWEGEPPCLPPRDSLN QSLSQDLTMAPGSTLWLSCGVPPDSVSRGPLSWTHVHPKGPKSLLSLELKDDRPARDMW VMETGLLLPRATAQDAGKYYCHRGNLTMSFHLEITARPVLWHWLLRTGGWKVSAVTLA YLIFCLCSLVGILHLQRALVLRRKRKRMTDPTRRFRGRGRGSLLTCGDVEENPGPMEFGLS WLFLVAILKGVQCSRDFVMTQSPSSLTVTAGEKVTMSCKSSQSLLNSGNQKNYLTWYLQ KPGQPPKLLIYWASTRESGVPDRFTGSGSGTDFTLTISSVQAEDLAVYYCQNDYSYPYTFG GGTKLEIKRGGGSGGGGEVQLQQSGPELVKPGASVKMSCKASGYTFTDYYMKWVKQSH GKSLEWIGDIIPSNGATFYNQKFKGKATLTVDRSSSTAYMHLNSLTSEDSAVYYCTRSHLL RASWFAYWGQGTLVTVSAGSELPTQGTFSNVSTNVSPAPRPPTPAPTIASQPLSLRPEACRP AAGGAVHTRGLDFACDIYIWAPLAGTCGVLLLSLVITLYCNHRNRRRVCKCPRVDKRGRK KLLYIFKQPFMRPVQTTQEEDGCSCRFPEEEEGGCELRVKFSRSADAPAYQQGQNQLYNE LNLGRREEYDVLDKRRGRDPEMGGKPRRKNPQEGLYNELQKDKMAEAYSEIGMKGERR RGKGHDGLYQGLSTATKDTYDALHMQALPPR*), more preferably it comprises or consists of SEQ ID NO:12.

Another object of the invetion is an isolated nucleic acid molecule encoding the chimeric antigen receptor molecule according to any one of the previous claims, wherein said isolated nuelcic acid molecule is preferably operatively linked to expression control sequences. Preferably said molecule comprises at least one sequence having at least 80% of identity to one the following sequence: SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26,
even more preferably said molecule comprises or consists of a sequence having at least 80% of identity to SEQ ID NO: 29.

A further object of the invention is a vector comprising the isolated nucleic acid molecule as above defined, preferably an expression vector, more preferably the vector comprises an exogenous promoter controlling the expression of the CAR, preferably said vector is a DNA, an RNA vector, a plasmid, a lentivirus vector, adenoviral vector, retrovirus vector or non-viral vector.

Another object of the invention is an engineered immune cell, preferably a T cell, more preferably an alfa/beta and/or gamma/delta T cell, or NK cells or NK-T cells or combinations thereof, even more preferably of human origin, comprising the vector as above defined or the isolated nucleic acid molecule as above defined or expressing, preferably at the cell surface, a CAR as above defined. More preferably, the cells are Innate cells.

A further object of the invention is a pharmaceutical composition comprising the the isolated nucleic acid molecule as above defined or the vector as above defined or the cell as above defined with at least one pharmaceutically acceptable excipiente and/or adjuvant.

Another object of the invention is a CAR molecule as above defined, the nucleotide sequence as above defined, the vector as above defined, the cell as above defined or the pharmaceutical composition as above defined for medical use, preferably for use in the teratment of CD123+ cancers, more preferably of acute myeloid or B-lymphoid leukemias, blastic plasmacytoid dendritic neoplasms (BPDCN), myelodispastic syndrome or hairy cell leukemia or for use before, after or during a haematopoietic stem cell transplantion.

Another object of the invention is the extracellular domain and transmembrane domain of human CD 19, in particular as defined above, for use as inducer of death in a cell genetically modified with said extracellular domain and transmembrane domain of human CD19, after the exposure of said cell to anti-CD19 antibody, including bite antibodies, e.g. Blinatumomab

The choice of the linker depends to the cloning strategy (insertion of a restriction enzyme).

Preferably, the above nucleic acid is integrated into the genome of the cell.

The CAR -cells may be allogeneic cells or autologous cells and/or HLA matched to the subject.

In the context of the present invention, the cancer preferably expresses CD123.

Preferably the polynucleotide is under the control of an endoogenous promoter.

It is another object of the invention a viral particle comprising the isolated nuelcic acid molecule as above defined or the vector as above defined.

The chimeric antigen receptors (CAR) of the invention may comprise an antibody or antibody fragment engineered for specific binding to a CD123 protein or fragments thereof. In one aspect, the antigen binding domain of the CAR comprises a human or humanized CD123 antibody or antibody fragment thereof. Preferably, the antigen binding domain of the CAR comprises human CD123 antibody fragment comprising an scFv, preferably a human CD123 scFv.

In one aspect, the CAR123 binding domain comprises the scFv portion provided in SEQ ID NO: 30

(DFVMTQSPSSLTVTAGEKVTMSCKSSQSLLNSGNQKNYLTWYLQKPGQPPKLLIYWAST
RESGVPDRFTGSGSGTDFTLTISSVQAEDLAVYYCQNDYSYPYTFGGGTKLEIKRGGGSGG
GGEVQLQQSGPELVKPGASVKMSCKASGYTFTDYYMKWVKQSHGKSLEWIGDIIPSNGA
TFYNQKFKGKATLTVDRSSSTAYMHLNSLTSEDSAVYYCTRSHLLRASWFAYWGQGTLV
TV).

The domains herein diclosed of the CAR are contiguous with and in the same reading frame to form a single fusion protein.

The cells of the invention (e.g., an immune effector cell, e.g., a T cell or a NK cell) may be engineered to express a CAR, wherein the CAR-expressing cell (e.g., "CAR-T" or CAR-expressing NK cell) exhibits an antitumor property. In one aspect a cell is transformed with the CAR and the at least part of the CAR is expressed on the cell surface. In some embodiments, the cell (e.g., immune effector cell, e.g., T cell or NK cell) is transduced with a viral vector encoding a CAR. In some embodiments, the viral vector is a retroviral vector. In some embodiments, the viral vector is a lentiviral vector. In some such embodiments, the cell may stably express the CAR. In another embodiment, the cell (e.g., immune effector cell, e.g., T cell or NK cell) is transfected with a nucleic acid, e.g., mRNA, cDNA, DNA, encoding a CAR. In some such embodiments, the cell may transiently express the CAR. Preferably, the CD123 binding domain, e.g., the human or humanized CD123 binding domain, of the CAR is a scFv antibody fragment. In one aspect, such antibody fragments are functional in that they retain the equivalent binding affinity, e.g., they bind the same antigen with comparable efficacy, as the IgG antibody having the same heavy and light chain variable regions. In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan. The antibodies of the invention may be incorporated into a chimeric antigen receptor (CAR). In one aspect, the CD123 binding domain, e.g., humanized or human CD123 binding domain, portion of a CAR of the invention is encoded by a transgene whose sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

"CD123" refers to an antigenic determinant known to be detectable on leukemia precursor cells. The human and murine amino acid and nucleic acid sequences can be found in a public database, such as GenBank, UniProt and Swiss-Prot. For example, the amino acid sequence of human CD123 can be found at Accession No. NP_002174.1 and the nucleotide sequence encoding of the human CD123 can be found at Accession No. NM_002183.4. In one aspect the antigen-binding portion of the CAR recognizes and binds an antigen within the extracellular domain of the CD 123 protein. In one aspect, the CD 123 protein is expressed on a cancer cell. As used herein, the term "Chimeric Antigen Receptor" or alternatively a "CAR" refers to a recombinant polypeptide construct comprising at least an antigen binding domain, a transmembrane domain and a cytoplasmic domain (also referred to herein as "an intracellular signaling domain") comprising a functional signaling domain derived from a stimulatory molecule as defined herein. In one aspect, the stimulatory molecule is the zeta chain associated with the T cell receptor complex. In one aspect, the intracellular signaling domain further comprises one or more functional signaling domains derived from at least one costimulatory molecule as defined below. In one aspect, the costimulatory molecule is chosen from 4-1BB (i.e., CD137), CD27, and/or CD28. In one aspect, the CAR comprises a chimeric fusion protein comprising an antigen recognition domain, a transmembrane domain and a cytoplasmic signaling domain comprising a functional signaling domain derived from a stimulatory molecule. In one aspect, the CAR comprises a chimeric fusion protein comprising an antigen recognition domain, a transmembrane domain and a cytoplasmic signaling domain comprising a functional signaling domain derived from a costimulatory molecule and a functional signaling domain derived from a stimulatory molecule. In one aspect, the CAR comprises a chimeric fusion protein comprising an antigen recognition domain, a transmembrane domain and an intracellular signaling domain comprising two functional signaling domains derived from one or more co-stimulatory molecule(s) and a functional signaling domain derived from a stimulatory molecule. In one aspect, the CAR comprises a chimeric fusion protein comprising an extracellular antigen recognition domain, a transmembrane domain and an intracellular signaling domain comprising at least two functional signaling domains derived from one or more costimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen recognition domain, wherein the leader sequence is optionally cleaved from the antigen recognition domain, e.g., a scFv) during cellular processing and localization of the CAR to the cellular membrane. As used herein, the terms intracellular, cytoplasmic and cytoplasmatic are used interchangeably. The term "signaling domain" refers to the functional portion of a protein which acts by transmitting information within the cell to regulate cellular activity via defined signaling pathways by generating second messengers or functioning as effectors by responding to such messengers. The term "antibody," as used herein, refers to a protein , or polypeptide sequence derived from an immunoglobulin molecule which specifically binds with an antigen, e.g., non-covalently, reversibly, and in a specific manner. An antibody can be polyclonal or monoclonal, multiple or single chain, or an intact immunoglobulin, and may be derived from natural sources or from recombinant sources. An antibody can be a tetramer of immunoglobulin molecule. For example, a naturally occurring IgG antibody is a tetramer comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CHI, CH2 and CH3. Each light chain is comprised of a light chain variable region (abbreviated herein as VL) and a light chain constant region. The light chain constant region is comprised of one domain, CL. The VH and VLregions can be further subdivided into regions of hyper variability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. The term "antibody" includes, but is not limited to, monoclonal antibodies, human antibodies, humanized antibodies, camelid antibodies, and chimeric antibodies. The antibodies can be of any isotype/class (e.g., IgG, IgE, IgM, IgD, IgA and IgY), or subclass (e.g., IgGl, IgG2, IgG3, IgG4, IgAl and IgA2). The term "antibody fragment" refers to at least one portion of an intact antibody, or recombinant variants thereof, and refers to the antigen binding domain, e.g., an antigenic determining variable regions of an intact antibody that is sufficient to confer recognition and specific binding of the antibody fragment to a target, such as an antigen. Examples of antibody fragments include, but are not limited to, Fab, Fab', F(ab')2, and Fv fragments, single chain or "scFv" antibody fragments, linear antibodies, single domain antbodies such as sdAb (either VLor VH), camelid VHHdomains, and multi- specific antibodies formed from antibody fragments. The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VLlinker-VH or may comprise VH-linker-VL. The portion of the CAR composition of the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv) and a humanized antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York) [20, 21]. In one aspect, the antigen binding domain of a CAR composition of the invention comprises an antibody fragment. In a further aspect, the CAR comprises an antibody fragment that comprises a scFv. By the term "recombinant antibody" as used herein, is meant an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant or synthetic DNA or amino acid sequence technology which is available and well known in the art. The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen.

The phrase "disease associated with expression of CD 123" as used herein includes but is not limited to, a disease associated with expression of CD 123 or condition associated with cells which express CD123 including, e.g., a proliferative disease such as a cancer or malignancy or a precancerous condition such as a myelodysplasia, a myelodysplasia syndrome or a preleukemia; or a noncancer related indication associated with cells which express CD123. In one aspect, a cancer associated with expression of CD 123 is a hematological cancer. In one aspect, a hematological cancer includes but is not limited to AML, myelodysplasia syndrome, ALL, hairy cell leukemia, Prolymphocytic leukemia, Chronic myeloid leukemia, Hodgkin lymphoma, Blastic plasmacytoid dendritic cell neoplasm, and the like. Further disease associated with expression of CD 123 expression include, but are not limited to, e.g., atypical and/or non- classical cancers, malignancies, precancerous conditions or proliferative diseases associated with expression of CD123. Non-cancer related indications associated with expression of CD 123 may also be included.

In the present invention, one or more amino acid residues within a CAR of the invention can be replaced with other amino acid residues from the same side chain family and the altered CAR can be tested for the ability to bind CD 123 using functional assays known to the skilled man.

A "stimulatory molecule," as the term is used herein, means a molecule expressed by a T cell that provides the primary cytoplasmic signaling sequence(s) that regulate primary activation of the TCR complex in a stimulatory way for at least some aspect of the T cell signaling pathway. In one aspect, the primary signal is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyro sine-based activation motif or ITAM. Examples of an IT AM containing primary cytoplasmic signaling sequence that is of particular use in the invention includes, but is not limited to, those derived from TCR zeta, FcR gamma, FcR beta, CD3 gamma, CD3 delta , CD3 epsilon, CD5, CD22, CD79a, CD79b, CD278 (also known as "ICOS") and CD66d. In a specific CAR molecule of the invention, the intracellular signaling domain in any one or more CAR molecules of the invention comprises an intracellular signaling sequence, e.g., a primary signaling sequence of CD3-zeta. In a specific CAR of the invention, the primary signaling sequence of CD3-zeta is the human sequence (SEQ ID NO:11), or the equivalent residues from a non-human species, e.g., mouse, rodent, monkey, ape and the like. In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule. A primary intracellular signaling domain can comprise a signaling motif which is known as an immunoreceptor tyrosine-based activation motif or IT AM. Examples of IT AM containing primary cytoplasmic signaling sequences include, but are not limited to, those derived from CD3 zeta, FcR gamma, FcR beta, CD3 gamma, CD3 delta, CD3 epsilon, CD5, CD22, CD79a, CD79b, and CD66d DAP10 and DAP12.

As used herein "zeta" or alternatively "zeta chain", "CD3-zeta" or "TCR-zeta" is defined as the protein provided as NCBI Acc. No. J04132.1, or the equivalent residues from a non-human species, e.g., mouse, rodent, monkey, ape and the like, and a "zeta stimulatory domain" or alternatively a "CD3-zeta stimulatory domain" or a "TCR- zeta stimulatory domain" is defined as the amino acid residues from the cytoplamic domain of the zeta chain that are sufficient to functionally transmit an initial signal necessary for T cell activation. A "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to, an MHC class I molecule, BTLA and a Toll ligand receptor, as well as OX40, CD27, CD28, CDS, ICAM-1, LFA-1 (CD11a/CD18) and 4-1BB (CD137). A costimulatory intracellular signaling domain can be derived from the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, , ICOS, BAFFR, HVEM, lymphocyte function-associated antigen- 1 (LFA-1), CD2, CD7, LIGHT, NKG2C, SLAMF7, NKp80, CD160, B7-H3, and a ligand that specifically binds with CD83, and the like. The intracellular signaling domain can comprise the entire intracellular portion, or the entire native intracellular signaling domain, of the molecule from which it is derived, or a functional fragment thereof. As used herein "4- IBB" refers to a member of the TNFR superfamily with an amino acid sequence provided as NCBI No. AAA53133.1, or the equivalent residues from a non-human species, e.g., mouse, rodent, monkey, ape and the like. In one aspect, the "4- IBB costimulatory domain" is the human sequence or the equivalent residues from a non-human species, e.g., mouse, rodent, monkey, ape and the like. In one aspect, the "4-1BB costimulatory domain" is the sequence provided as SEQ ID NO: 10 or the equivalent residues from a non-human species, e.g., mouse, rodent, monkey, ape and the like. "Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g. rRNA, tRNA and mRNA) or a defined sequence of amino acids and the biological properties resulting therefrom. Thus, a gene, cDNA, or RNA encodes a protein if transcription and translation of mRNA corresponding to that gene, cDNA, or RNA produces the protein in a cell or other biological system. Both the coding strand, the nucleotide sequence of which is identical to the mRNA sequence, and the non-coding strand, used as the template for transcription of a gene or cDNA, can be referred to as encoding the protein or other product of that gene or cDNA. Unless otherwise specified, a "nucleotide sequence encoding an amino acid sequence" includes all nucleotide sequences that are degenerate versions of each other and that encode the same amino acid sequence. Nucleotide sequences that encode proteins or a RNA may also include introns to the extent that the nucleotide sequence encoding the protein may in some version contain an intron(s). The term "vector" or "transfer vector" refers to a polynucleotide which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to further include non- plasmid and non-viral compounds which facilitate transfer of nucleic acid into cells, such as, for example, a polylysine compound, a liposome, and the like. Examples of viral vectors include, but are not limited to, adenoviral vectors, adeno-associated virus vectors, retroviral vectors, lentiviral vectors, and the like. "Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g. , naked or contained in liposomes) and viruses (e.g. , lentiviruses, retroviruses, adenoviruses, and adeno-associated viruses) that incorporate the recombinant polynucleotide. The term "homologous" or "identity" as used herein, refers to the subunit sequence identity between two polymeric molecules, e.g. , between two nucleic acid molecules, such as, two DNA molecules or two RNA molecules, or between two polypeptide molecules. When a subunit position in both of the two molecules is occupied by the same monomeric subunit; e.g. , if a position in each of two DNA molecules is occupied by adenine, then they are homologous or identical at that position. The homology between two sequences is a direct function of the number of matching or homologous positions; e.g. , if half (e.g. , five positions in a polymer ten subunits in length) of the positions in two sequences are homologous, the two sequences are 50% homologous; if 90% of the positions (e.g. , 9 of 10), are matched or homologous, the two sequences are 90% homologous.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

A "lentiviral vector" is a vector derived from at least a portion of a lentivirus genome, including especially a self-inactivating lentiviral vector as provided in Milone et al [22]. Other Examples or lentivirus vectors that may be used in the clinic include but are not limited to, e.g., the LENTIVECTOR® gene delivery technology from Oxford BioMedica, the LENTIMAX™ vector system from Lentigen and the like. Nonclinical types of lentiviral vectors are also available and would be known to one skilled in the art.

The term "operably linked" or alternatively "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame. The term "nucleic acid" or "polynucleotide" refers to deoxyribonucleic acids (DNA) or ribonucleic acids (RNA) and polymers thereof in either single- or double- stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues [23] [24] [25]. As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" (as the above domain, marker, peptide, linker,...) include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, a synthetic peptide, or a combination thereof. The term "promoter" as used herein is defined as a DNA sequence recognized by the synthetic machinery of the cell, or introduced synthetic machinery, required to initiate the specific transcription of a polynucleotide sequence. As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner. A "linker" as used in the context of an scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser-Gly-Gly-Gly-Gly)n (SEQ ID NO:27), where n is a positive integer equal to or greater than 1. For example, n=1, n=2, n=3. n=4, n=5 and n=6, n=7, n=8, n=9 and n=10. Also included within the scope of the invention are linkers described in WO2012/138475, incorporated herein by reference in its entirety. The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny. As used herein, "transient" refers to expression of a non-integrated transgene for a period of hours, days or weeks, wherein the period of time of expression is less than the period of time for expression of the gene if integrated into the genome or contained within a stable plasmid replicon in the host cell.The phrase "under transcriptional control" or "operatively linked" as used herein means that the promoter is in the correct location and orientation in relation to a polynucleotide to control the initiation of transcription by RNA polymerase and expression of the polynucleotide. By the term "specifically binds," as used herein, is meant an antibody or antigen binding fragment thereof, or a ligand, which recognizes and binds with a cognate binding partner (e.g. a stimulatory and/or costimulatory molecule present on a T cell) protein present in a sample, but which antibody, antigen binding fragment thereof or ligand does not substantially recognize or bind other molecules in the sample. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range. The term at least 80% identity, includes something with 80%, 81%, 82, 83%, 84%, 85%, 86%, 87%, 88%, 89% , 90%, 91%, 92%, 93%, 94%, 95%, 96%, 97%, 98% 99% or 100%identity.

Included in the present invention are also nucleic acid and amino acid sequences derived from the sequences shown above, e.g. functional fragments, mutants, derivatives, analogues, and sequences having a % of identity of at least 70% with the above sequences.

The invention will be illustrated by means of non-limiting examples in reference to the following figures.
**Figure 1****: Design of the ΔCD19-2A-CAR.CD123-ΔCD34-CD8.4.1BB-ζ (CAR.CD123-T) retroviral vector**
   The expression cassette of **CAR.CD123** shown in cartoon. The bicistronic vector is composed by two transgenes, namely ΔCD19 and CARCD123. ΔCD19 consists of (extracellular and transmembrane) domains of the CD19 marker. It is connected to CARCD123 by 2A peptide.
   The scFv of CD123 was cloned in frame with CD8a spacer-transmembrane domain and a costimulatory domain represented by 4-1BB, as well as the signaling domain CD3-zeta chain (ζ). As a trackable marker, ΔCD34 was added.
**Figure 2****: Level of transduction of CAR-CD123 vector in T cells.**
   a) Flow-cytometry analyses of the CD3/CD34 expression in T cells un-transduced (NT-T)(CD3+CD34-)(left panel) or genetically modified with ΔCD19-2A-CAR.CD123-ΔCD34-CD8.4.1BB-ζ (CAR.CD123-T) (CD3+CD34+) (right panel). The experiment represents T cells from an exemplificative donor, growth in IL7/IL15, and then transduced or not with the novel vector.
   b) T cells from three donors genetically modified with CAR.CD123-T. The histogram represents the average of the percentage of CD3+CD34+ cells profiled by FACS at two time points *of in vitro* expansion ± SD.
**Figure 3****: T cells fold expansion overtime.**
   T cells un-transduced (NT-T) or genetically modified with ΔCD19-2A-CAR.CD123-ΔCD34-CD8.4.1BB-ζ (CAR.CD123-T) were counted on a weekly base to monitor cell expansion. Each dot is the average number of T cells from three donors ± SD.
**Figure 4****: Level of transduction of CAR-CD123 vector in Innate Cells** .
   a) Flow-cytometry analyses of the CD56/CD34 expression in Innate Cells un-transduced (NT-Innate Cells )(CD56+CD34)(left panel) or genetically modified with ΔCD19-2A-CAR.CD123-ΔCD34-CD8.4.1BB-ζ (CAR.CD123- Innate Cells)(CD56+CD34+)(right panel). The experiment represents Innate Cells from an exemplificative donor, transduced or not with the novel vector.
   b) Innate Cells from three donors genetically modified with ΔCD19-2A-CAR.CD123-4.1BB-ζ. The histogram represents the average of the percentage of CD56+CD34+ cells profiled by FACS at two time points of in vitro expansion ± SD.

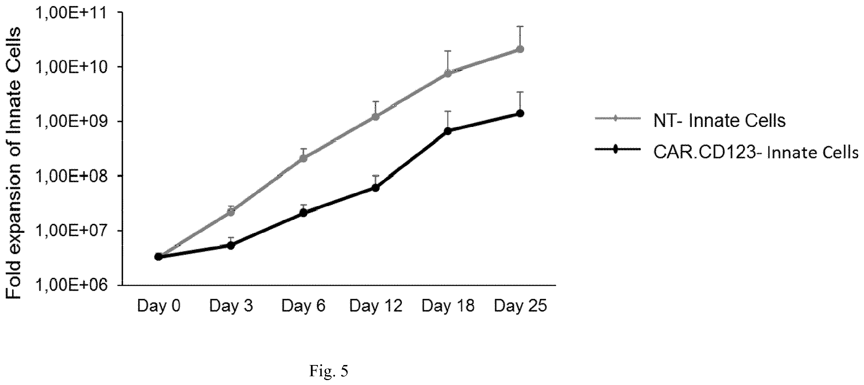

**Figure 5****: Innate cells fold expansion overtime.**
   Innate Cells un-transduced (NT- Innate Cells) or genetically modified with ΔCD19-2A-CAR.CD123-ΔCD34-CD8.4.1BB-ζ (CAR.CD123- Innate Cells) were counted on a weekly base to monitor cell expansion. Each dot is the average number of Innate Cells from three donors ± SD.

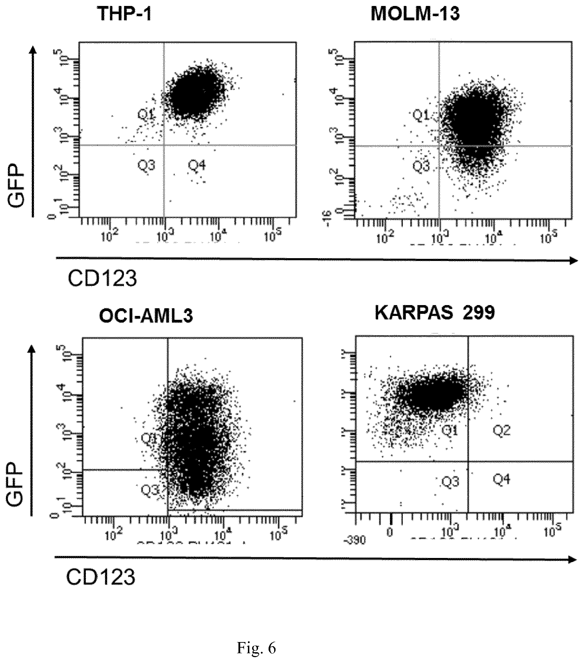

**Figure 6****: Phenotypic characterization of CD123 expression in leukemia and lymphoma cell lines.** FACS analysis of CD123 expression in three leukemia cell lines (THP1, MOLM13, OCI AML3) and a lymphoma cell line (KARPAS 299) shows that the CD123 expression is specific to leukemia cells.

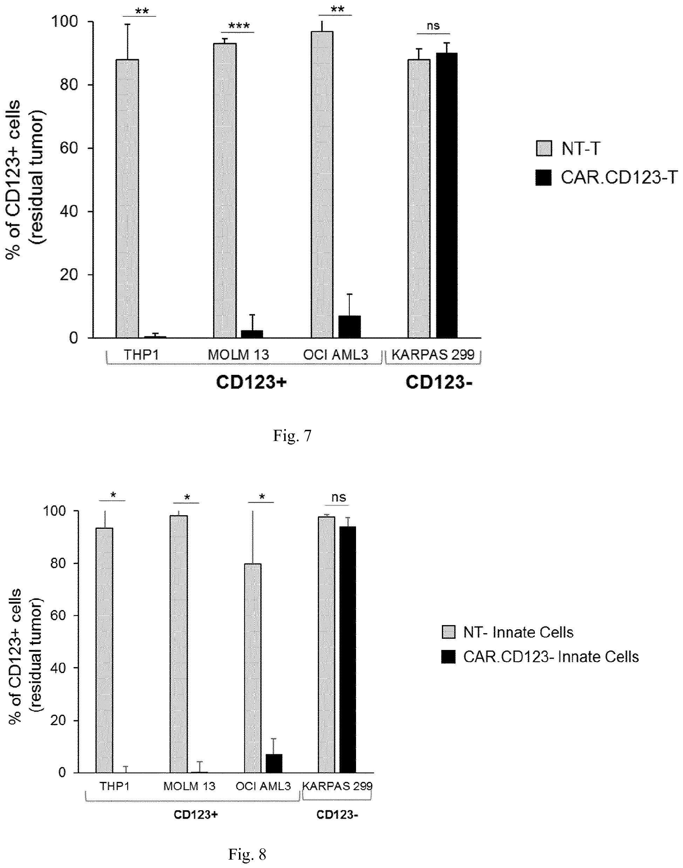

**Figure 7****: Long-term anti-tumor activity of CAR.CD123- T cells.** Three CD123+ cell lines (THP1, MOLM13, OCI AML3) and CD123- cells (KARPAS 299) have been co-cultured with un-transduced T cells (NT-T) or T cells genetically modified with ΔCD19-2A-CAR.CD123-ΔCD34-CD8.4.1BB-ζ (CAR.CD123-T). The histogram shows the % of remaining tumor cells after 5 days-coculture at the ratio 1:1 with either NT-T (light gray) or CAR.CD123-T cells (black bar). The experiment was performed in triplicate with T cells coming from three donors. The results are expressed as average of the biological replicates ± SD. ** p-value≤0.01; *** p-value<0.001; ns=not significant.
**Figure 8****: Long-term anti-tumor activity of CAR.CD123 Innate cells**. Three CD123+ cell lines (THP1, MOLM3, OCI AML3) and CD123- cells (KARPAS 299) have been co-cultured with un-transduced Innate Cells (NT- Innate Cells ) or Innate Cells genetically modified with ΔCD19-2A-CAR.CD123-ΔCD34-CD8.4.1BB-ζ (CAR.CD123- Innate Cells ). The histograms show the % of remaining tumor cells after 5 days-coculture at the ratio 1:1 with either NT- Innate Cells (light gray) or CAR.CD123- Innate Cells (black bar). The experiment was performed in triplicate with Innate cells coming from three donors. The results are expressed as average of the biological replicates ± SD. * p-value<0.05; ns=not significant.

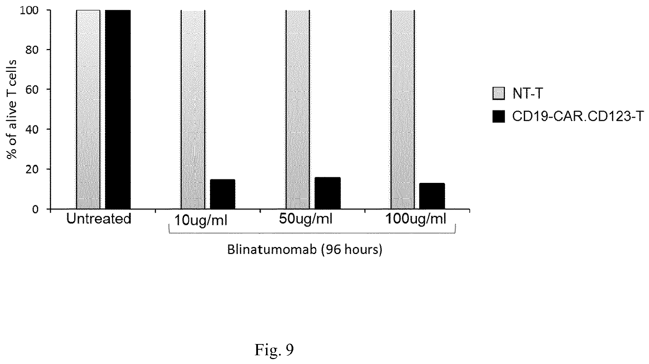

**Figure 9****: Effect of CD19 activation on T cell survival.** Flow-cytometry analysis of live T cells upon CD19 selective activation with Blinatumomab. T cells from an exemplificative donor were un-transduced (NT-T) (light grey) or genetically modified with ΔCD19-2A-CAR.CD123-ΔCD34-CD8.4.1BB-ζ (black), growth in IL7/IL15 -/+ Blinatumomab. The histogram shows the T cell survival after 96 hours of treatment.

### MATERIAL AND METHODS

### Design of CAR-CD123 plasmid (Constructs)

A clinical grade "second" generation of retrovirus bicistronic vector SFG have been designed, allowing the simultaneous expression of two transgenes, namely ΔCD19 and the cassette anti-CD123 single-chain variable fragment (scFv), derived from a murine antibody of IgG (7G3) class, linked via a codon optimized human CD8 spacer-transmembrane domain, to the codon optimized signaling costimulatory domain 4-1BB (CD137) and CD3-ζ (Figure 1).

In particular a ΔCD19 represents the extracellular domain of human CD19 linked to the transmembrane portion. The single chain variable fragment (scFv)- specific for CD123- is a fusion protein of 114 amino acid (aa) of the variable regions of the light chains (VL) of immunoglobulins connected by flex (a short linker peptide) of 8 amino acids to 118 aa of heavy chains (VH) of immunoglobulins.

In particular, the scFv 7G3 is cloned in frame with codon optimized CD34 derived epitope of 16 aa (as trackable marker), linked by spacer of 42 aa (11 aa as spacer plus 31 aa of codon optimized CD8 extracellular domain) to bind the codon optimized human CD8-transmembrane domain (CD8aTM) of 21aa. The signal run from extracellular portion of CD123 scFv 7G3 to intracellular portion of CD3-ζ chain (113aa) through costimulatory molecule: 4-1BB endodomain (42aa) for the SFG: **ΔCD19-2A-CAR.CD123-ΔCD34-CD8.4.1BB-ζ (CAR.CD123-T) retroviral vector.**

### Generation of eGFP-Firefly-Luciferase cell lines.

The retroviral vector encoding eGFP-Firefly-Luciferase (eGFP-FFLuc) was used in selected experiments to label CD123 positive (CD123⁺) or CD123 negative (CD123⁻) tumor cells:
CD123⁺:
   - Acute Monocytic Leukemia cell line THP-1
   - Acute Myeloid Leukemia cell line MOLM-13
   - Acute Myeloid Leukemia cell line OCI-AML-3
CD123⁻:
   - Non-Hodgkin's Lymphoma (NHL) Karpas 299

### Cell lines.

Acute Monocytic Leukemia cell line THP-1 cell was obtained from LGC Standards-ATCC. Acute Myeloid Leukemia cell line MOLM-13 and OCI-AML-3 were obtained from DSMZ. Non-Hodgkin's Lymphoma (NHL) Karpas 299 was obtained from Sigma-Aldrich.

The THP-1, MOLM-13 and the Karpas 299 cell lines were maintained in culture with RPMI 1640 medium. The OCI-AML-3 were maintained in culture with IMDM. Cell lines were supplemented with 10% fetal bovine serum (FBS, Hyclone, Thermo Scientific, Pittsburgh, PA) and 2 mM GlutaMax (Invitrogen, California, USA). Cells were maintained a humidified atmosphere containing 5% CO2 at 37°C. All cell lines were routinely tested for mycoplasma and for surface expression of target antigen CD123. All cell lines have been authenticated by STR analysis in the certificated lab "BMR Genomics s.r.1."

### Retroviral supernatant

Transient retroviral supernatant was produced by cotransfection of 293T with the MoMLV gag/pol expression plasmid PeqPam3(-env), the RD114 env expression plasmid RDF, and SFG vectors at a ratio of 2:3:3, respectively, with a total of 10 µg DNA. The transfection was facilitated with GeneJuice reagent (Calbiochem). The supernatant was harvested 2 and 3 days after transfection, filtered (using a 0.45-mm filter), snap-frozen, and stored at -80°C in 5-ml aliquots [26].

### Isolation, generation and transduction of effector cells.

Peripheral blood mononuclear cells (PBMC) were isolated from peripheral blood (PB) or buffy coat obtained from healthy donors (OPBG Hospital, Rome, Italy) after that signed informed consent was obtained, in accordance with rules set by Institutional Review Board (IRB) of OPBG (Approval of Ethical Committee N°969/2015 prot. N° 669LB), using Lymphocytes separation medium (Eurobio; France). T lymphocytes were activated with immobilized OKT3 (1 µg/ml, e-Bioscience Inc.;San Diego,CA, USA) and anti-CD28 (1 µg/ml, BD Biosciences, Europe) antibodies in the presence of combination of recombinant human interleukin-7 (IL7, 10 ng/ml; R&D; USA) [27] and recombinant human interleukin-15 (IL15, 5 ng/ml; R&D) [28] [29]. Activated T cells were transduced on day 3 in 24-well plates pre-coated with recombinant human RetroNectin (Takara-Bio. Inc; Japan) using a specific retroviral supernatant and the specific above-described cytokines. At day 5 from transduction the T cells are expanded in "CTL complete medium" containing 45% RPMI1640 and 45% Click's medium (Sigma-Aldrich,Co.; Usa) supplemented with 10% FBS and 2 mM Glutamax, and fed twice a week with the specific above described cytokines [30].

### Isolation, generation and transduction of Innate cells

Peripheral blood mononuclear cells (PBMC) were isolated from healthy donor's buffy coat or leukapheresis by a density-gradient technique (Ficool-Histopaque (Eurobio;France); the healthy donors had signed a written informed consent, in accordance with rules set by the Institutional Review Board of OPBG (Approval of Ethical Committee N_969/2015 prot. N_669LB). CD56+ CD3- NK cells, isolated with an NK isolation Kit (Miltenyi Biotec, Inc., San Diego, CA, USA), and expanded with NK Cell Activation/Expansion Kit (Miltenyi Biotec, Inc., San Diego, CA, USA) and recombinant human interleukin 2 (IL2, 500 U/ml; R&D; USA) or recombinant interleukin 15 (IL15 10 U/ml; R&D; USA). Activated NK cells were transduced in 24-well plates pre-coated with recombinant human RetroNectin (Takara-Bio. Inc; Japan) using retroviral supernatant. Enriched NK cells were cultured in GMP-compliant media (NK MACS Miltenyi Biotec, Inc., San Diego, CA, USA).

To obtain Innate Cells (NK cells and/or gamma/delta T cells) Alpha-beta depleted cells were expanded in NK MACS media and fed twice a week with the specific above described cytokines. Activate Innate cell combining both populations were transduced in 24-well plates pre-coated with recombinant human RetroNectin (Takara-Bio. Inc; Japan) using retroviral supernatant

### Phenotypic analysis

The following mAbs were used: CD3, CD123, CD45, CD56, CD34, CD19, (All from BD Pharmigen, USA). The expression of CAR.CD123 on T cells was evaluated using anti-CD34 Ab (R&D, USA) or the Pierce Recombinant Biotinylated Protein L [31] (Thermo Fisher Scientific, USA). Samples were analyzed with a BD LSRFortessa X-20 and Diva software (BD Biosciences). For each sample, a minimum of 20,000 events have been analyzed.

### Fold expansion

Cells were count one time for week by Counting Chambers using a trypan blue method.

### In vitro anti-leukemia activity

For co-culture experiments, effector T lymphocytes and leukemia cell lines were plated in 24-well plates. Following 5 days of incubation at 37°C, tumor cells and T cells were collected and analyzed by FACS.

### Induction of apoptosis of CAR-123 Cells

The bispecific antibody against human CD19 human CD3 (Aurogene) was added at the indicated concentrations to the NT or CAR.CD123 T cells (Innate.NT or Innate.CAR.123). The elimination of genetically modified CAR+ cells were evaluated at 72 hours by FACS analysis of viable cells.

### Statistical analysis

Statistical evaluation was performed using GraphPad Prism (GraphPad Software). Differences between groups generating p-values <0.05 were considered significantly.

### RESULTS

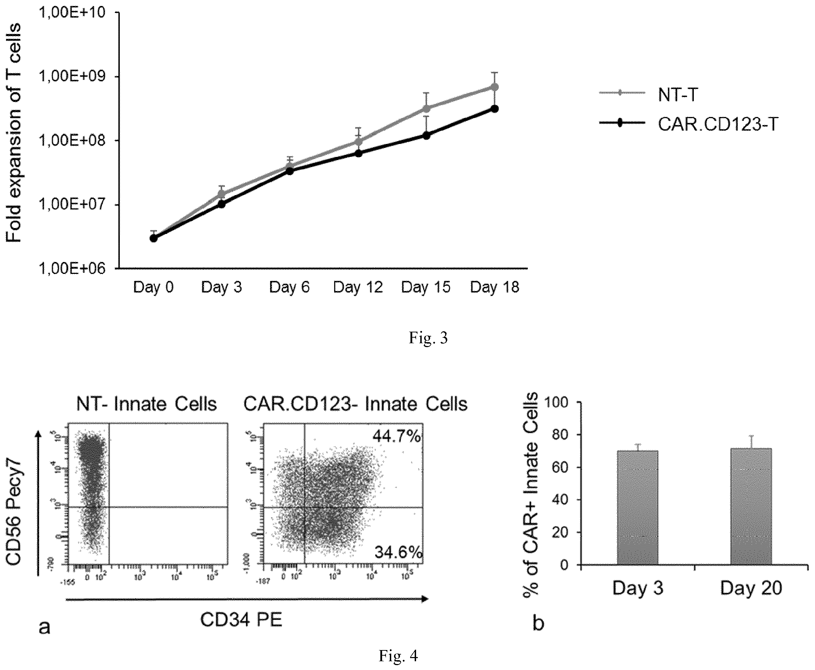

The CAR.CD123-4.1BB-ζ sequence mentioned above according to the present invention provides unexpected advantages in comparison with the known CARs-CD123 (Table 2) such as:
the introduction of ΔCD19 as selectable marker and inducible suicide gene (Figure 9);
furthermore, inventors show a high level of stable CAR-CD123 expression in both T cells and in Innate cells (NK cells or and gamma/delta T cells) (Figures 2 and 4).

The in vitro results herewith described show that modified polyclonal ΔCD19-2A-CAR.CD123-4.1BB-ζ T cells/ Innate cells, according to the present invention were able to eliminate very efficiently, in long-term co-culture, CD123+ tumors (Figures 6, 7 and 8).

More in detail, the supernatants obtained by SFG retroviral vector were able to transduce efficiently activated T cells (Figure 2) or Innate Cells (Figure 4), with very high level of transduction. Furthermore, the retroviral vector expression is stable over time in both T cells (Figure 2b) or Innate Cells (Figure 4b) without interfering with the cell expansion (Figure 3 and 5). The introduction in the construct of CD34 derived epitope as trackable marker let easily to track the genetically modified T cells (CD3+CD34+) and NK cells (CD56+CD34+) in vitro model (Figures 2a and 4a).

### REFERENCES

1. Shi, M., et al., CD123 a novel biomaker for diagnosis and treatment of leukemia. Cardiovasc Hematol Disord Drug Targets, 2019.
2. Sapienza, M.R., et al., Elastic Plasmacytoid Dendritic Cell Neoplasm: State of the Art and Prospects. Cancers (Basel), 2019. 11(5).
3. Stevens, B.M., et al., CD123 CAR T cells for the treatment of myelodysplastic syndrome. Exp Hematol, 2019. 74: p. 52-63 e3.
4. Salem, D.A., et al., Differential Expression of CD43, CD81, and CD200 in Classic Versus Variant Hairy Cell Leukemia. Cytometry B Clin Cytom, 2019.
5. Forman, S.J. and J.M. Rowe, The myth of the second remission of acute leukemia in the adult. Blood, 2013.121(7): p. 1077-82.
6. Majeti, R., Monoclonal antibody therapy directed against human acute myeloid leukemia stem cells. Oncogene, 2011. 30(9): p. 1009-19.
7. Korpelainen, E.I., et al., Interferon-gamma upregulates interleukin-3 (IL-3) receptor expression in human endothelial cells and synergizes with IL-3 in stimulating major histocompatibility complex class II expression and cytokine production. Blood, 1995. 86(1): p. 176-82.
8. Li, K., et al., Expression of complement components, receptors and regulators by human dendritic cells. Mol Immunol, 2011. 48(9-10): p. 1121-7.
9. Taussig, D.C., et al., Hematopoietic stem cells express multiple myeloid markers: implications for the origin and targeted therapy of acute myeloid leukemia. Blood, 2005. 106(13): p. 4086-92.
10. Munoz, L., et al., Interleukin-3 receptor alpha chain (CD123) is widely expressed in hematologic malignancies. Haematologica, 2001. 86(12): p. 1261-9.
11. Testa, U., et al., Elevated expression of IL-3Ralpha in acute myelogenous leukemia is associated with enhanced blast proliferation, increased cellularity, and poor prognosis. Blood, 2002. 100(8): p. 2980-8.
12. Graf, M., et al., Expression and prognostic value of hemopoietic cytokine receptors in acute myeloid leukemia (AML): implications for future therapeutical strategies. Eur J Haematol, 2004. 72(2): p. 89-106.
13. Vergez, F., et al., High levels of CD34+CD38low/-CD123+ blasts are predictive of an adverse outcome in acute myeloid leukemia: a Groupe Ouest-Est des Leucemies Aigues et Maladies du Sang (GOELAMS) study. Haematologica, 2011. 96(12): p. 1792-8.
14. Tettamanti, S., et al., Targeting of acute myeloid leukaemia by cytokine-induced killer cells redirected with a novel CD123-specific chimeric antigen receptor. Br J Haematol, 2013. 161(3): p. 389-401.
15. Smith, B.D., et al., First-in Man, Phase 1 Study of CSL362 (Anti-IL3Rα / Anti-CD123 Monoclonal Antibody) in Patients with CD123+ Acute Myeloid Leukemia (AML) in CR at High Risk for Early Relapse. Blood, 2014. 124(21): p. 120-120.
16. Testa, U., E. Pelosi, and A. Frankel, CD 123 is a membrane biomarker and a therapeutic target in hematologic malignancies. Biomark Res, 2014. 2(1): p. 4.
17. Mardiros, A., et al., T cells expressing CD123-specific chimeric antigen receptors exhibit specific cytolytic effector functions and antitumor effects against human acute myeloid leukemia. Blood, 2013. 122(18): p. 3138-48.
18. Gill, S., et al., Preclinical targeting of human acute myeloid leukemia and myeloablation using chimeric antigen receptor-modified T cells. Blood, 2014. 123(15): p. 2343-54.
19. Thokala, R., et al., Redirecting Specificity of T cells Using the Sleeping Beauty System to Express Chimeric Antigen Receptors by Mix-and-Matching of VL and VH Domains Targeting CD123+ Tumors. PLoS One, 2016. 11(8): p. e0159477.
20. Huston, J.S., et al., Protein engineering of antibody binding sites: recovery of specific activity in an anti-digoxin single-chain Fv analogue produced in Escherichia coli. Proc Natl Acad Sci U S A, 1988. 85(16): p. 5879-83.
21. Bird, R.E., et al., Single-chain antigen-binding proteins. Science, 1988. 242(4877): p. 423-6.
22. Milone, M.C., et al., Chimeric receptors containing CD137 signal transduction domains mediate enhanced survival of T cells and increased antileukemic efficacy in vivo. Mol Ther, 2009. 17(8): p. 1453-64.
23. Batzer, M.A., J.E. Carlton, and P.L. Deininger, Enhanced evolutionary PCR using oligonucleotides with inosine at the 3'-terminus. Nucleic Acids Res, 1991. 19(18): p. 5081.
24. Ohtsuka, E., et al., An alternative approach to deoxyoligonucleotides as hybridization probes by insertion of deoxyinosine at ambiguous codon positions. J Biol Chem, 1985. 260(5): p. 2605-8.
25. Rossolini, G.M., et al., Use of deoxyinosine-containing primers vs degenerate primers for polymerase chain reaction based on ambiguous sequence information. Mol Cell Probes, 1994. 8(2): p. 91-8.
26. Pule, M.A., et al., A chimeric T cell antigen receptor that augments cytokine release and supports clonal expansion of primary human T cells. Mol Ther, 2005. 12(5): p. 933-41.
27. Perna, S.K., et al., Interleukin-7 mediates selective expansion of tumor-redirected cytotoxic T lymphocytes (CTLs) without enhancement of regulatory T-cell inhibition. Clin Cancer Res, 2014. 20(1): p. 131-9.
28. Perna, S.K., et al., Interleukin 15 provides relief to CTLs from regulatory T cell-mediated inhibition: implications for adoptive T cell-based therapies for lymphoma. Clin Cancer Res, 2013.19(1): p. 106-17.
29. Cieri, N., et al., IL-7 and IL-15 instruct the generation of human memory stem T cells from naive precursors. Blood, 2013. 121(4): p. 573-84.
30. Zheng, Z., N. Chinnasamy, and R.A. Morgan, Protein L: a novel reagent for the detection of chimeric antigen receptor (CAR) expression by flow cytometry. J Transl Med, 2012. 10: p. 29.
31. Klement, M., et al., Effect of linker flexibility and length on the functionality of a cytotoxic engineered antibody fragment. J Biotechnol, 2015. 199: p. 90-7.

## Claims

1. A chimeric antigen receptor (CAR) molecule comprising, from the N-terminus to the C-terminus:
a) an extracellular domain and transmembrane domain of human CD19,
b) an antigen binding domain,
c) a spacer domain,
d) a transmembrane domain,
e) a cytoplasmatic domain,
preferably wherein the CAR is a CD123 specific CAR (CD123 CAR) and the antigen binding domain comprises VH and/or VL from a monoclonal anti-CD123 antibody, more preferably the antigen binding domain comprises a single chain variable fragment (scFv) that specifically binds to CD123.

2. The CAR molecule according to claim 1 wherein the extracellular and transmembrane domains of human CD19 comprises or consist of a sequence having at least 80% of identity to SEQ ID NO: 1 (MPPPRLLFFLLFLTPMEVRPEEPLVVKVEEGDNAVLQCLKGTSDGPTQQLTWSRESPLKPF LKLSLGLPGLGIHMRPLAIWLFIFNVSQQMGGFYLCQPGPPSEKAWQPGWTVNVEGSGEL FRWNVSDLGGLGCGLKNRSSEGPSSPSGKLMSPKLYVWAKDRPEIWEGEPPCLPPRDSLN QSLSQDLTMAPGSTLWLSCGVPPDSVSRGPLSWTHVHPKGPKSLLSLELKDDRPARDMW VMETGLLLPRATAQDAGKYYCHRGNLTMSFHLEITARPVLWHWLLRTGGWKVSAVTLA YLIFCLCSLVGILHLQRALVLRRKRKRMTDPTRRF), preferably it comprises or consists of SEQ ID NO:1.

3. The CAR molecule according to claim 1 or 2 wherein the the antigen binding domain comprises comprises a sequence having at least 80% of identity to the 7G3 VL sequence: DFVMTQSPSSLTVTAGEKVTMSCKSSQSLLNSGNQKNYLTWYLQKPGQPPKLLIYWASTR ESGVPDRFTGSGSGTDFTLTISSVQAEDLAVYYCQNDYSYPYTFGGGTKLEIKR (SEQ ID NO:4) and/or a sequence having at least 80% of identity to the 7G3: EVQLQQSGPELVKPGASVKMSCKASGYTFTDYYMKWVKQSHGKSLEWIGDIIPSNGATFY NQKFKGKATLTVDRSSSTAYMHLNSLTSEDSAVYYCTRSHLLRASWFAYWGQGTLVTV( SEQ ID NO:5), said VH and/or VL being optionally humanized, wherein said VH and VL are preferably separated by a first linker, more preferably said scFv comprises a VL comprising SEQ ID NO: 4 and a VH comprising SEQ ID NO: 5 .

4. The CAR molecule according to any one of previous claims wherein the spacer comprises the extacellular region of the CD8 alpha chain, preferably it comprises or consists of a sequence having at least 80% of identity to aa. 12-42 of SEQ ID NO: 7 (IASQPLSLRPEACRPAAGGAVHTRGLDFACD) and/or the spacer comprises or consists of a sequence having at least 80% of identity to aa. 1-11 of SEQ ID NO:7 (PAPRPPTPAPT), more preferably the spacer comprisises SEQ ID NO:7.

5. The CAR molecule according to any one of previous claims wherein the trans membrane domain comprises a trasmembrane domain of a protein selected from the group consiting of CD8, alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD15, preferably it comprises the trans membrane domain of a CD8, more preferably it comprises or consists of a sequence having at least 80% of identity to SEQ ID NO: 8 (IYIWAPLAGTCGVLLLSLVIT), even more preferably it comprises SEQ ID NO: 8.

6. The CAR molecule according to any one of previous claims, wherein said cytoplasmatic domain comprises a region of CD8a cytoplasmatic portion, a 4-1BB co-stimulatory domain and a CD3-zeta chain, preferably said cytoplasmatic domain comprises a linker between the CD8a cytoplasmic and the 4-1BB co-stimulatory domain, more preferably:
- the CD8a cytomplasmic comprises or consistis of a sequence having at least 80% of identity to SEQ ID NO:9 (LYCNHRNRRRVCKCPR), even more preferably it comprises SEQ ID NO:9, and/or
- the co-stimulatory signalling domain CD137 (4-1BB) comprises or consistis of a sequence having at least 80% of identity to SEQ ID NO:10 (KRGRKKLLYIFKQPFMRPVQTTQEEDGCSCRFPEEEEGGCEL), even more preferably it comprises SEQ ID NO: 10, and/or
- the CD3-Zeta chain comprises or consistis of a sequence having at least 80% of identity to SEQ ID NO: 11 (RVKFSRSADAPAYQQGQNQLYNELNLGRREEYDVLDKRRGRDPEMGGKPRRKNPQEGL YNELQKDKMAEAYSEIGMKGERRRGKGHDGLYQGLSTATKTDTYDALHMQALPPR*), even more preferably it comprises SEQ ID NO:11, and/or
- the sequence encoding the linker between the CD8a cytomplasmic and the co-stimulatory signalling domain CD137 (4-1BB) consists of 6 nucleotides.

7. The CAR molecule according to any one of previous claims furter comprising:
i. between the extracellular domain and transmembrane domain of human CD19 and the antigen binding domain:
- a clevable linker, preferably a 2A peptide or an IRES, and/or
- a signal peptide and/or
- a linker, and/or
ii. between the antigen binding domain and the spacer domain.
- a linker and/or
- a trackable marker,
preferably:
- the 2 A peptide comprises or consists of a sequence having at least 80% of identity to SEQ ID NO:2 (RGRGRGSLLTCGDVEENPGP), more preferably it comprises SEQ ID NO:2, and/or
- the signal peptide comprises or consist of a sequence having at least 80% of identity to SEQ ID NO:3 (MEFGLSWLFLVAILKGVQC), more preferably it comprises SEQ ID NO:3, and/or
- the trackable marker comprises or consist of a sequence having at least 80% of identity to SEQ ID NO:6 (ELPTQGTFSNVSTNVS), more preferably it comprises SEQ ID NO:6.

8. The CAR molecule according to any one of previous claims wherein the sequence encoding for at least one of: the extracellular domain and transmembrane domain of human CD19, the trackable marker, the spacer domain, the transmembrane domain and the co-stimulatory domain is codon optimized.

9. The CAR molecule according to any one of previous claims wherein said molecule comprises:
from the N-terminus to the C-terminus:
a) an extracellular domain and transmembrane domain of human CD19, as defined in any one of claim 1-2 and 8,
b) a clevable linker, as defined in claim 7,
c) a signal peptide, as defined in claim 7,
d) an antigen binding domain, as defined in any one of claims 1 and 3
e) a trackable marker, as defined in claims 7-8.
f) a spacer domain, as defined in any one of claims 1 and 4 and 8
g) a transmembrane domain, as defined in any one of claims 1 and 5 and 8
h) a cytoplasmatic domain, as defined in any one of claims 1 and 6 and 8,
preferably the CAR molecule comprises or consists of a sequence having at least 80% of identity to SEQ ID NO:12, more preferably it comprises a sequence of SEQ ID NO:12.

10. An isolated nucleic acid molecule encoding the chimeric antigen receptor molecule according to any one of the previous claims, wherein said isolated nuelcic acid molecule is preferably operatively linked to expression control sequences, more preferably said molecule comprises at least one sequence having at least 80% of identity to one the following sequence: SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26,
even more preferably said molecule comprises or consists of a sequence having at least 80% of identity to SEQ ID NO: 29.

11. A vector comprising the isolated nucleic acid molecule according to claim 10, preferably an expression vector, more preferably the vector comprises an exogenous promoter controlling the expression of the CAR, preferably said vector is a DNA, an RNA vector, a plasmid, a lentivirus vector, adenoviral vector, retrovirus vector or non-viral vector.

12. An engineered immune cell, preferably a T cell, more preferably an alfa/beta and gamma/delta T cell, or NK cells or NK-T cells or combinations thereof, even more preferably of human origin, comprising the vector according to claim 11 or the isolated nucleic acid molecule according to claim 10 or expressing at the cell surface a CAR according to any one of claims 1-9.

13. A pharmaceutical composition comprising the the isolated nucleic acid molecule according to claim 10 or the vector according to claim 11 or the cell according to claim 12 with at least one pharmaceutically acceptable excipiente and/or adjuvant.

14. The CAR molecule according to any one of claims 1-9, the isolated nucleic acid molecule according to claim 10, the vector according to claim 11, the cell according to claim 12 or the pharmaceutical composition of claim 13 for medical use, preferably for use in the teratment of CD123+ cancers, more preferably of acute myeloid or B-lymphoid leukemias, blastic plasmacytoid dendritic neoplasms (BPDCN), myelodispastic syndrome or hairy cell leukemia or for use before, after or during a haematopoietic stem cell transplantion.

15. The extracellular domain and transmembrane domain of human CD19 as defined in any one of claims 1-2 and 8, for use as inducer of death in a cell genetically modified with said extracellular domain and transmembrane domain of human CD 19, after the exposure of said cell to anti-CD 19 antibody, including bite antibodies, e.g. Blinatumomab.
